(19) Europäisches Patentamt
European Patent Office
Office européen des brevets

(11) Veröffentlichungsnummer: **0 507 824 B1**

(12) # EUROPÄISCHE PATENTSCHRIFT

(45) Veröffentlichungstag der Patentschrift: **19.10.94**

(51) Int. Cl.[5]: **C07C 233/36**, C07C 233/65, C07D 241/08, C07D 295/16, C07D 471/04, C07D 487/04

(21) Anmeldenummer: **91901789.7**

(22) Anmeldetag: **17.12.90**

(86) Internationale Anmeldenummer:
**PCT/EP90/02199**

(87) Internationale Veröffentlichungsnummer:
**WO 91/09835 (11.07.91 91/15)**

(54) **BISKATIONISCHE SÄUREAMID- UND -IMIDDERIVATE UND VERFAHREN ZU IHRER HERSTELLUNG.**

(30) Priorität: **28.12.89 DE 3943047**
**05.09.90 DE 4028121**

(43) Veröffentlichungstag der Anmeldung:
**14.10.92 Patentblatt 92/42**

(45) Bekanntmachung des Hinweises auf die Patenterteilung:
**19.10.94 Patentblatt 94/42**

(84) Benannte Vertragsstaaten:
**CH DE FR GB IT LI NL**

(56) Entgegenhaltungen:
**EP-A- 0 026 157**
**EP-A- 0 122 324**
**EP-A- 0 125 475**
**EP-A- 0 327 379**
**GB-A-21 123 88**
**US-A- 4 299 982**

**PATENT ABSTRACTS OF JAPAN, Band 9, Nr. 52, C269, Zusammenfassung von JP 59-190976**

(73) Patentinhaber: **HOECHST AKTIENGESELLSCHAFT**

**D-65926 Frankfurt (DE)**

(72) Erfinder: **MACHOLDT, Hans-Tobias**
**Waldstrasse 20**
**D-6100 Darmstadt (DE)**
Erfinder: **SCHIESSLER, Siegfried**
**Rother Weingartenweg 48**
**D-6232 Bad Soden am Taunus (DE)**
Erfinder: **GITZEL, Jörg**
**Domherrnstrasse 2**
**D-6234 Hattersheim am Main (DE)**
Erfinder: **DIETZ, Erwin**
**St.-Matthäus-Strasse 7**
**D-6233 Kelkheim (Taunus) (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der vorliegenden Erfindung sind neue biskationische Säureamid- und -imidderivate und Verfahren zu ihrer Herstellung. Die vorliegende Erfindung betrifft im einzelnen neue biskationische Säureamid- und -imidderivate der allgemeinen Formeln (I) bis (III):

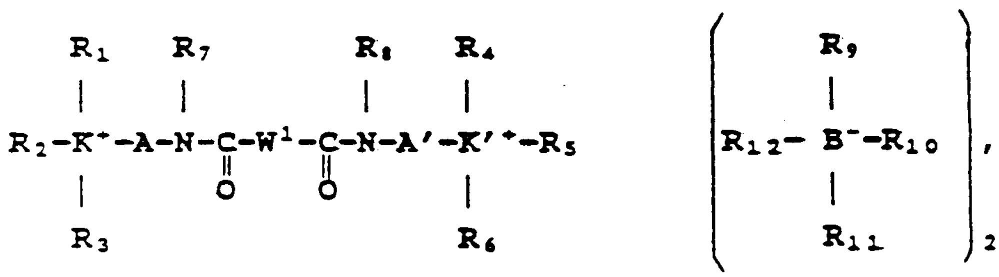

(I)

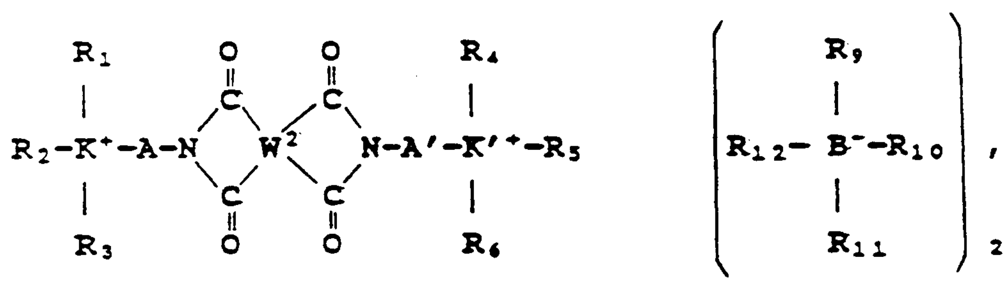

(II)

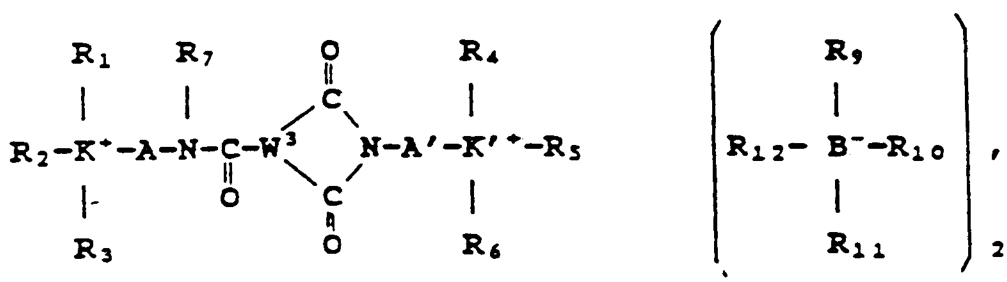

(III)

wobei $R_1$ bis $R_8$ unabhängig voneinander jeweils ein Wasserstoffatom oder einen Rest auf Basis eines Kohlenwasserstoffes, wie z. B. eine geradkettige oder verzweigte, gesättigte oder ungesättigte Alkylgruppe mit 1 bis 30 Kohlenstoffatomen, vorzugsweise mit 1 bis 22 Kohlenstoffatomen, eine Polyoxalkylengruppe der allgemeinen Formel $-[Alkylen(C_1-C_5)-O]_n-R$, worin R ein Wasserstoffatom oder eine Alkyl $(C_1-C_4)$-gruppe, eine Acylgruppe, wie beispielsweise eine Acetyl-, Benzoyl- oder Naphthoylgruppe, und n eine Zahl von 1 bis 10, vorzugsweise von 1 bis 4, bedeutet, einen ein- oder mehrkernigen cycloaliphatischen Rest von 5 bis 12 Kohlenstoffatomen, wie beispielsweise einen Cyclopentyl- oder Cyclohexylrest, einen ein- oder mehrkernigen aromatischen Rest, wie beispielsweise einen Phenyl-, Naphthyl-, Tolyl- oder Biphenylrest, oder einen araliphatischen Rest, wie beispielsweise einen Benzylrest, darstellen, wobei die genannten aliphatischen, cyloaliphatischen, araliphatischen oder aromatischen Reste durch Carbon- oder Sulfonsäuregruppen, deren Salze bzw. Amide oder Ester, Alkyl$(C_1-C_4)$-, Hydroxy-, Alkoxy$(C_1-C_4)$gruppen, primäre, sekundäre oder tertiäre Aminogruppen, wie beipielsweise N-Monoalkyl$(C_1-C_4)$amino- oder N-Dialkyl$(C_1-C_4)$-

aminogruppen, sowie durch Fluor-, Chlor- oder Bromatome, die aliphatischen Reste bevorzugt durch 1 bis 45 Fluoratome, substituiert sein können, und die genannten aliphatischen Reste sowie die cycloaliphatischen, araliphatischen oder aromatischen Ringsysteme ein oder mehrere Heteroatome, wie beipielsweise Stickstoff- und/oder Sauerstoff- und/oder Schwefel- und/oder Phosphoratome, enthalten können, und $R_1$ und $R_2$ bzw. $R_4$ und $R_5$ sich unabhängig voneinander unter Einbeziehung von K bzw. K' Zu einem gesättigten oder ungesättigten, bevorzugt aromatischen, 5- bis 7-gliedrigen Ringsystem zusammenschließen können, das weitere Heteroatome, bevorzugt Stickstoff- und/oder Sauerstoff- und/oder Schwefelatome, enthalten sowie substituiert und/oder durch Ankondensation von oder Verbrückung zu weiteren Ringsystemen modifiziert sein kann, und wobei für den Fall, daß $R_1$ oder $R_2$ bzw. $R_4$ oder $R_5$ in diesem Zusammenhang eine Doppelbindung zu K bzw. K' ausbilden, $R_3$ und $R_6$ gegenstandslos sind, und/oder einer der Reste $R_1$, $R_2$ oder $R_3$ sich mit $R_7$ bzw. einer der Reste $R_4$, $R_5$ oder $R_6$ sich mit $R_8$ zu einer aliphatischen Brücke aus 2 bis 5 Kohlenstoffatomen zusammenschließen kann,

und A und A' unabhängig voneinander jeweils ein Brückenglied auf der Basis eines Kohlenwasserstoffes, wie z. B. ein geradkettiges oder verzweigtes, gesättigtes oder ungesättigtes Alkylenbrückenglied von 1 bis 30 Kohlenstoffatomen, vorzugsweise von 1 bis 12 Kohlenstoffatomen, ein ein- oder mehrkerniges cycloaliphatisches Brückenglied, wie beispielsweise ein Cyclopentylen- oder Cyclohexylenglied, ein ein- oder mehrkerniges aromatisches Brückenglied, wie beispielsweise ein Phenylen-, Naphthylen-, Tolylen- oder Biphenylenglied, oder araliphatisches Brückenglied, wie beispielsweise ein Benzylen-, Xylylen-, Mesitylen- oder Benzoylenamidglied, darstellen, wobei die aliphatischen, cycloaliphatischen, araliphatischen oder aromatischen Brückenglieder durch Carbon- oder Sulfonsäuregruppen, deren Salze bzw. Amide oder Ester, Alkyl($C_1$-$C_4$)-, Hydroxy-, Alkoxy($C_1$-$C_4$)gruppen, primäre, sekundäre oder tertiäre Aminogruppen, wie beipielsweise N-Monoalkyl($C_1$-$C_4$)amino- oder N-Dialkyl($C_1$-$C_4$)aminogruppen, sowie durch Fluor-, Chlor- oder Bromatome, die aliphatischen Reste bevorzugt durch 1 bis 45 Fluoratome substituiert sein können, und die genannten aliphatischen, cycloaliphatischen, araliphatischen oder aromatischen Brückenglieder ein oder mehrere Heteroatome, wie beispielsweise Stickstoff- und/oder Sauerstoff- und/oder Schwefel- und/oder Phosphoratome, enthalten können,

und $W^1$ als zweiwertiges Brückenglied ein geradkettiges oder verzweigtes, gesättigtes oder ungesättigtes aliphatisches Brückenglied mit 1 bis 30 Kohlenstoffatomen, bevorzugt mit 1 bis 22 Kohlenstoffatomen, ein Polyoxalkylenglied, bevorzugt ein Polyoxethylen- oder Polyoxyropylenglied, der allgemeinen Formel $-CH_2-[Alkylen(C_1-C_5)-O]_m-CH_2-$, wobei m eine Zahl von 0 bis 10, vorzugsweise von 1 bis 4, ist, ein ein- oder mehrkerniges cycloaliphatisches Brückenglied mit 5 bis 12 Kohlenstoffatomen, wie beispielsweise ein Cyclopentylen- oder Cyclohexylenglied, ein ein- oder mehrkerniges aromatisches Brückenglied, wie beispielsweise ein Phenylen-, Naphthylen-, Tolylen- oder Biphenylenglied, ein araliphatisches Brückenglied, wie beispielsweise ein Benzylenglied, darstellt, wobei die aliphatischen, cycloaliphatischen, araliphatischen und aromatischen Brückenglieder durch Carbon- oder Sulfonsäuregruppen, deren Salze bzw. Amide oder Ester, Alkyl($C_1$-$C_4$)-, Hydroxy-, Alkoxy($C_1$-$C_4$)gruppen, primäre, sekundäre oder tertiäre Aminogruppen, wie beipielsweise N-Monoalkyl($C_1$-$C_4$)amino- oder N-Dialkyl($C_1$-$C_4$)aminogruppen, sowie durch Fluor-, Chlor- oder Bromatome, die aliphatischen Reste bevorzugt durch 1 bis 45 Fluoratome substituiert sein können, und die genannten aliphatischen, cycloaliphatischen, araliphatischen oder aromatischen Brückenglieder ein oder mehrere Heteroatome, wie beipielsweise Stickstoff- und/oder Sauerstoff- und/oder Schwefel- und/oder Phosphoratome, enthalten können, und $W^1$ aber auch eine direkte Bindung bedeuten kann,

und $W^2$ als vierwertiges Brückenglied ein geradkettiges oder verzweigtes, gesättigtes oder ungesättigtes aliphatisches Brückenglied mit 2 bis 30 Kohlenstoffatomen, bevorzugt 2 bis 12 Kohlenstoffatomen, ein ein- oder mehrkerniges cycloaliphatisches Brückenglied mit 5 bis 12 Kohlenstoffatomen, wie beispielsweise Cyclopentylen- oder Cyclohexylenglied, ein ein- oder mehrkerniges aromatisches Brückenglied, wie beispielsweise ein Phenylen-, Naphthylen-, Tolylen- oder Biphenylenglied, ein araliphatisches Brückenglied, wie beispielsweise ein Benzylenglied, darstellt, wobei die aliphatischen, cycloaliphatischen, araliphatischen und aromatischen Brückenglieder durch Carbon- oder Sulfonsäuregruppen, deren Salze bzw. Amide oder Ester, Alkyl($C_1$-$C_4$)-, Hydroxy-, Alkoxy($C_1$-$C_4$)gruppen, primäre, sekundäre oder tertiäre Aminogruppen, wie beipielsweise N-Monoalkyl($C_1$-$C_4$)amino- oder N-Dialkyl($C_1$-$C_4$)aminogruppen, sowie durch Fluor-, Chlor- oder Bromatome, die aliphatischen Reste bevorzugt durch 1 bis 45 Fluoratome, substituiert sein können, und die genannten aliphatischen, cycloaliphatischen, araliphatischen oder aromatischen Brückenglieder ein oder mehrere Heteroatome, wie beipielsweise Stickstoff- und/oder Sauerstoff- und/oder Schwefel- und/oder Phosphoratome, enthalten können,

und $W^3$ als dreiwertiges Brückenglied ein geradkettiges oder verzweigtes, gesättigtes oder ungesättigtes aliphatisches Brückenglied mit 2 bis 30 Kohlenstoffatomen, bevorzugt 2 bis 12 Kohlenstoffatomen, ein ein- oder mehrkerniges cycloaliphatisches Brückenglied mit 5 bis 12 Kohlenstoffatomen, wie beispielsweise ein Cyclopentylen- oder Cyclohexylenglied, ein ein- oder mehrkerniges aromatisches Brückenglied, wie beispielsweise ein Phenylen-, Naphthylen-, Tolylen- oder Biphenylenglied, ein araliphatisches Brückenglied, wie beispielsweise ein Benzylenglied, darstellt, wobei die aliphatischen, cycloaliphatischen, araliphatischen und aromatischen Brückenglieder durch Carbon- oder Sulfonsäuregruppen, deren Salze bzw. Amide oder Ester, Alkyl($C_1$-$C_4$)-, Hydroxy-, Alkoxy($C_1$-$C_4$)gruppen, primäre, sekundäre oder tertiäre Aminogruppen, wie beipielsweise N-Monoalkyl($C_1$-$C_4$)amino- oder N-Dialkyl($C_1$-$C_4$)aminogruppen, sowie durch Fluor-, Chlor- oder Bromatome, die aliphatischen Reste bevorzugt durch 1 bis 45 Fluoratome, substituiert sein können, und die genannten aliphatischen, cycloaliphatischen, araliphatischen oder aromatischen Brückenglieder ein oder mehrere Heteroatome, wie beipielsweise Stickstoff- und/oder Sauerstoff- und/oder Schwefel- und/oder Phosphoratome, enthalten können,
und K bzw. K' unabhängig voneinander ein Stickstoff-, Phosphor-, Arsen- oder Antimonatom, bevorzugt ein Stickstoffatom, ist
und die Reste $R_9$ bis $R_{12}$ unabhängig voneinander aliphatische Reste, wie beipielsweise Methyl-, Ethyl-, Propyl-, Decyl- oder Stearylreste, cylcoaliphatische Reste, wie beispielsweise Cyclopentyl- oder Cyclohexylreste, iso- oder heterocyclische aromatische Reste, wie beispielsweise Phenyl-, Tolyl-, Naphthyl- oder Pyridylreste, Aralkylreste, wie beispielsweise Benzylreste, wobei die genannten aliphatischen, cycloaliphatischen, araliphatischen und aromatischen Reste durch Alkyl($C_1$-$C_4$)-, Alkoxy($C_1$-$C_4$)-, Arylreste oder Halogenatome substituiert sein können, oder Fluoratome bedeuten sowie Gemische dieser Verbindungen und Mischkristalle mit gemischten Anionen und/oder Kationen.
Die Erfindung betrifft insbesondere solche Verbindungen der oben genannten allgemeinen Formeln (I) bis (III), in denen $R_1$ bis $R_6$ unabhängig voneinander jeweils ein Wasserstoffatom, eine geradkettige oder verzweigte Alkyl($C_1$-$C_6$)gruppe, wie beispielsweise eine Methyl-, Ethyl-, n-Propyl-, iso-Propyl-, tertiär-Butyl-, Pentyl- oder Hexylgruppe, bedeuten und solche, in denen unabhängig voneinander $R_1$ und $R_2$ bzw. $R_4$ und $R_5$ unter Einbeziehung von K bzw. K',
wobei K und K' Stickstoffatome bedeuten,
sich zu einem gesättigten oder ungesättigten heterocyclischen Ringsystem mit einem oder mehreren Stickstoffatom(en) als Heteroatom, wie beispielsweise Pyrrol, Pyrrolin, Pyrrolidin, Pyrazol, Pyrazolon, Pyrazolin, Imidazol, Oxazol, Thiazol, Triazol, Pyridin, Piperidin, Pyrazin, Piperazin, Pyrimidin, Hexamethylenimin oder Morpholin, zusammenschließen können, wobei diese Ringsysteme wiederum substituiert sein können, bevorzugt mit geradkettigen Alkyl($C_1$-$C_4$)gruppen, und/oder durch Ankondensation von oder Verbrückung zu weiteren Ringsystemen vergrößert sein können, wie beispielsweise zu Chinolin, Indol, Indolin, Purin, Chinoxalin, Benzthiazol, Acridin, Benzchinolin, Carbazol, Benzophenazin, Phenanthrolin, Bipiperidin, Bipyridin, Phenazin, Benzacridin oder Nicotin, wobei $R_3$ und/oder $R_6$ jeweils ein Wasserstoffatom oder eine Alkyl($C_1$-$C_4$)gruppe darstellen, und für den Fall, daß $R_1$ oder $R_2$ bzw. $R_4$ oder $R_5$ eine Doppelbindung zu K oder K' ausbilden $R_3$ bzw.$R_6$ dann gegenstandslos sind,
$R_7$ und $R_8$ ein Wasserstoffatom bedeuten,
A und A' unabhängig voneinander ein $-CH=C(COOH)-CH_2-$,
$-CH_2-(CH_2-CH=CH-)_n-$ oder $-(CH_2-)_n$-Brückenglied mit n = 1 bis 12, bevorzugt mit n = 1 bis 4, ein Phenylen-, Naphthylen- oder - $-CO-NH-(CH_2)_3$-Brückenglied,
$W^1$ ein Phenylen-, Naphthylen-, Cyclohexylen-,
$-(CH_2-)_q$-Brückenglied mit q = 1 bis 12 oder ein $-CH_2-O-(CH_2-CH_2-O)_r-CH_2$-Brückenglied mit r = 1 bis 4,
$W^2$ ein Ethylendiamintetramethylen-, Phenylen- oder Naphthylen-Brückenglied, wobei die zur Imidbindung benötigten Carboxylgruppen im Falle des Phenylengliedes jeweils in ortho-Stellung, im Falle des Naphthylengliedes in ortho- und/oder peri-Stellung zueinander stehen,
und $W^3$ ein Phenylen- oder Naphthylen-Brückenglied bedeuten, wobei mindestens zwei an der Imidbindung beteiligten Carboxylgruppen sich in ortho- oder peri-Stellung zueinander befinden und die dritte Carboxylgruppe sich in beliebiger Stellung dazu befinden kann,
und für A und A' sowie $W^1$ als Phenylen-Brückenglieder in 1,2-, 1,3-, 1,4-Stellung, bevorzugt in 1,3- und 1,4-Stellung, als Naphthylen-Brückenglieder in 1,2- bis 1,8- sowie in 2,3- bis 2,8-Stellung und die Cyclohexylen-Brückenglieder in 1,2-, 1,3-, 1,4-Stellung, bevorzugt in 1,3- und 1,4-Stellung, zu dem jeweiligen K bzw. K' auf der einen und Imid- oder Amidstickstoff auf der anderen Seite verbrückt bzw. von den jeweiligen Carbonylfunktionen substituiert sind,
und die Reste $R_9$ bis $R_{12}$ Phenyl-, Naphthyl-, Fluorphenyl-, Chlorphenyl-, Methoxyphenyl-, Biphenyl-, Pyridyl-, Tolylreste oder Fluoratome bedeuten sowie Gemische dieser Verbindungen und Mischkristalle mit gemischten Anionen und/oder Kationen.

An Einzelverbindungen der oben genannten allgemeinen Formeln (I) bis (III) seien beispielsweise folgende genannt:

| Verbindung | Kation | Anion |
|---|---|---|
| 1.1a | 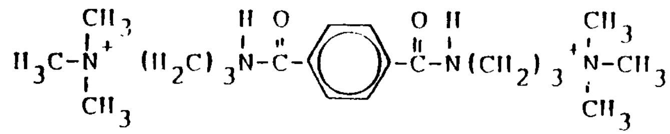 | $2BF_4^-$ |
| 1.1b | s.o. | 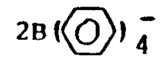 |
| 1.1c | s.o. | 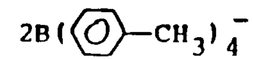 |
| 1.1d | s.o. | 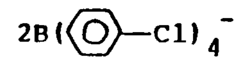 |
| 1.1e | s.o. | 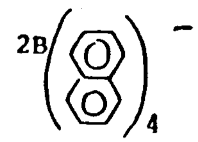 |
| 1.2a | 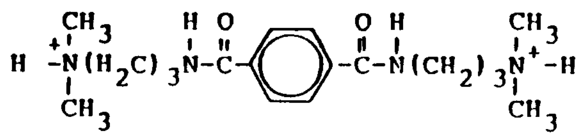 | $2BF_4^-$ |
| 1.2b | s.o. | 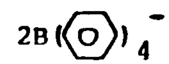 |
| 1.2c | s.o. | 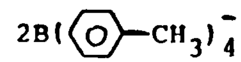 |
| 1.2d | s.o | 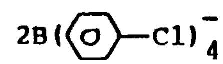 |
| 1.2e | s.o. | 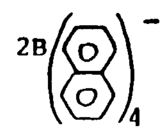 |

| Verbindung | Kation | Anion |
|---|---|---|
| 1.3a | 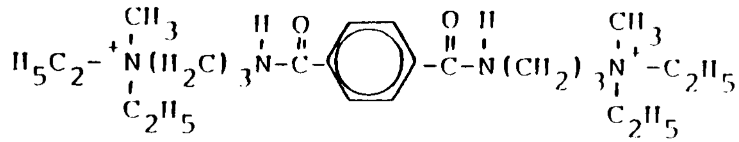 | $2BF_4^-$ |
| 1.3b | s.o. | 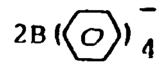 |
| 1.4a | 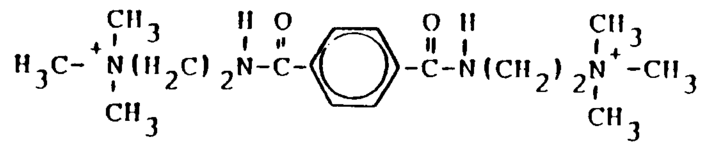 | $2BF_4^-$ |
| 1.4b | s.o. | 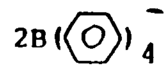 |
| 1.5a | 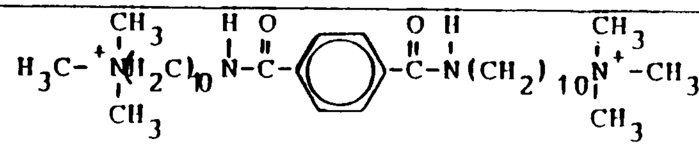 | $2BF_4^-$ |
| 1.5b | s.o. | 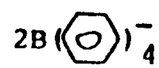 |
| 1.6a | 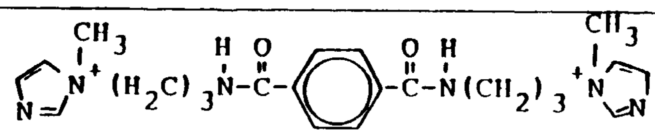 | $2BF_4^-$ |
| 1.6b | s.o. | 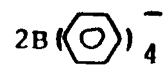 |

| Verbindung | Kation | Anion |
| --- | --- | --- |
| 1.7a | 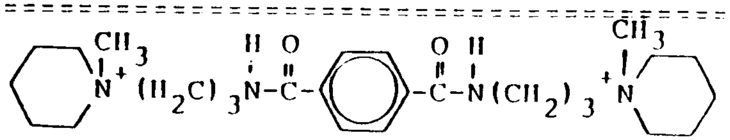 | $2BF_4^-$ |
| 1.7b | s.o. | 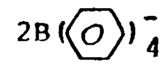 |
| 1.8a | 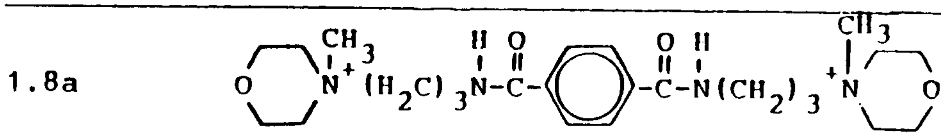 | $2BF_4^-$ |
| 1.8b | s.o. | 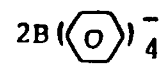 |
| 1.9a | 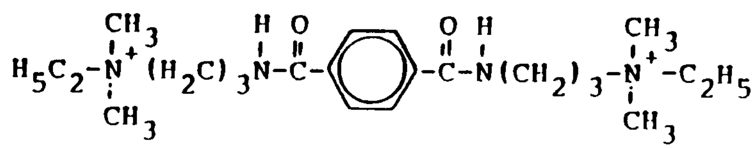 | $2BF_4^-$ |
| 1.9b | s.o. | 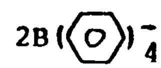 |

| Verbindung | Kation | Anion |
|---|---|---|
| 1.10a | 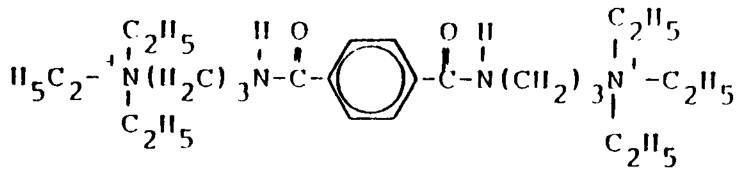 | $2BF_4^-$ |
| 1.10b | s.o. | 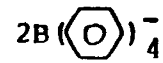 |
| 1.11a | 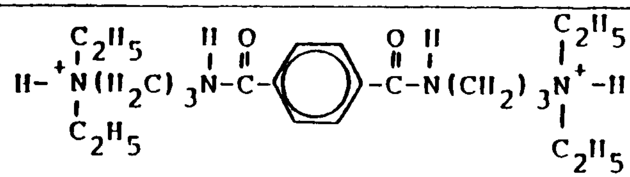 | $2BF_4^-$ |
| 1.11b | s.o. | 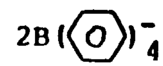 |
| 1.11c | s.o. | 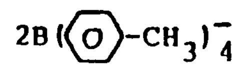 |
| 1.11d | s.o. | 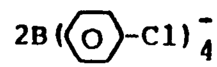 |
| 1.11e | s.o. | 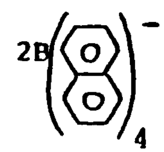 |

| Verbindung | | Kation | Anion |
|---|---|---|---|
| 2.1a | | 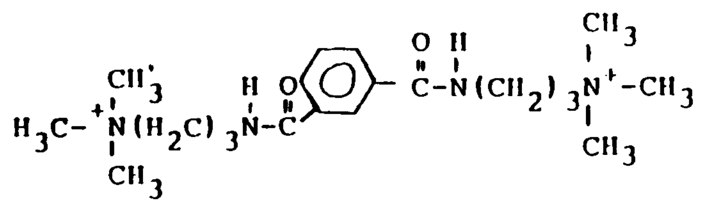 | $2BF_4^-$ |
| 2.1b | | s.o. | 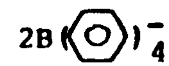 |
| 3.1a | cis/trans | 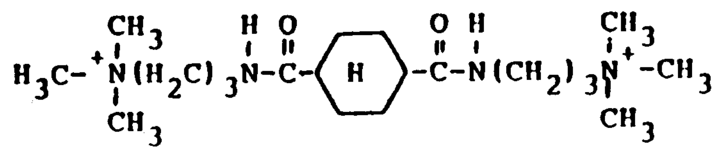 | $2BF_4^-$ |
| 3.1b | | s.o. | 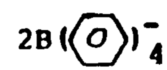 |
| 3.1c | | s.o. | 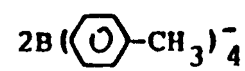 |
| 3.1d | | s.o. | 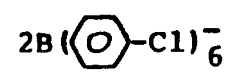 |
| 3.1e | | s.o. | 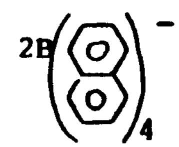 |

| Verbindung | | Kation | Anion |
|---|---|---|---|
| 3.2a | cis/trans | 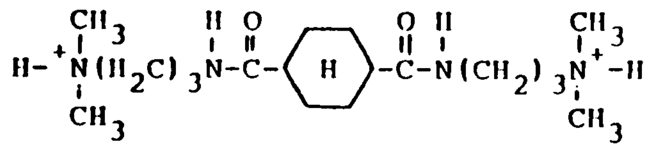 | 2-$BF_4^-$ |
| 3.2b | | s.o. | 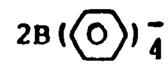 |
| 3.2c | | s.o. | 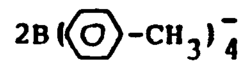 |
| 3.2d | | s.o. | 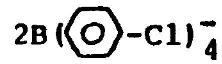 |
| 3.2e | | s.o. | 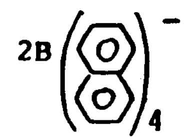 |
| 3.3a | cis/trans | 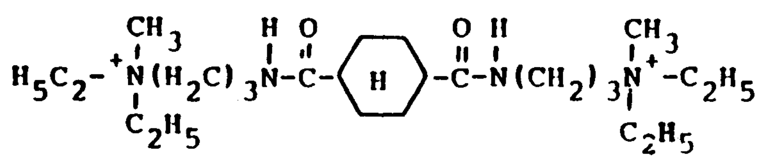 | 2$BF_4^-$ |
| 3.3b | | s.o. | 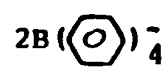 |

| Verbindung | Kation | Anion |
|---|---|---|
| 3.4a | 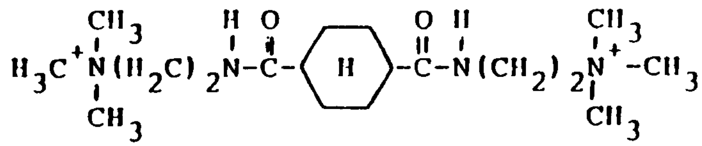 | $2BF_4^-$ |
| 3.4b | s.o. | 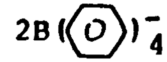 |
| 3.5a | 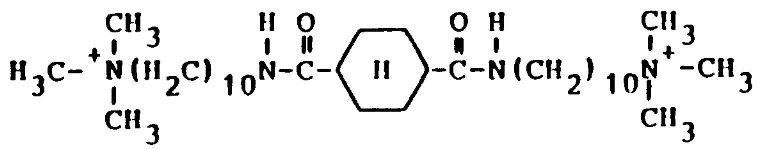 | $2BF_4^-$ |
| 3.5b | s.o. | 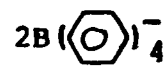 |
| 4.1a | 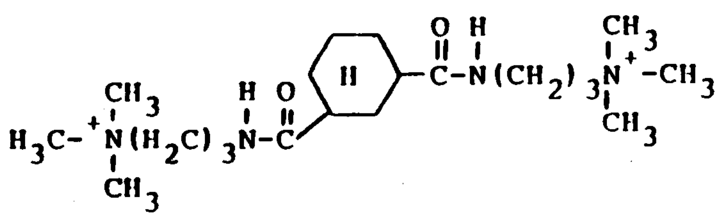 | $2BF_4^-$ |
| 4.1b | s.o. | 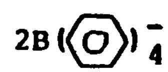 |

| Verbindung | Kation | Anion |
| --- | --- | --- |
| 5.1a | 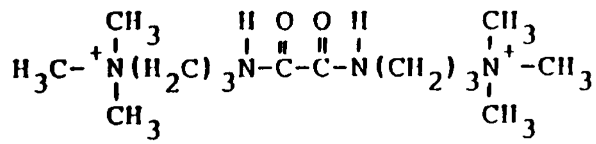 | $2BF_4^-$ |
| 5.1b | s.o. | 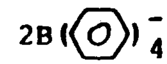 |
| 6.1a | 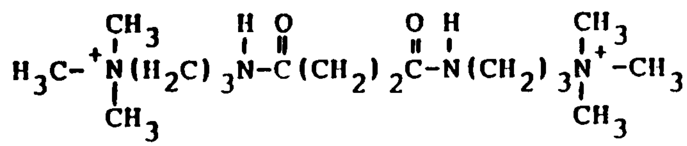 | $2BF_4^-$ |
| 6.1b | s.o. | 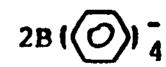 |
| 6.1c | s.o. | 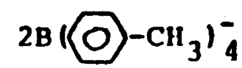 |
| 6.1d | s.o. | 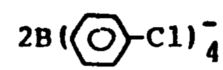 |
| 6.1e | s.o. | 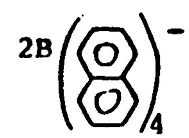 |

| Verbindung | Kation | Anion |
|---|---|---|
| 6.2a | 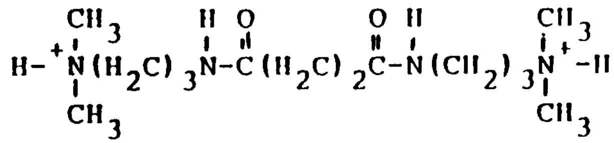 | $2BF_4^-$ |
| 6.2b | s.o. | 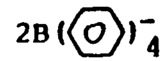 |
| 6.2c | s.o. | 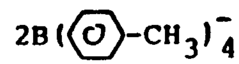 |
| 6.2d | s.o. | 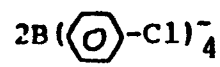 |
| 6.2e | s.o. | 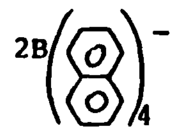 |
| 6.3a | 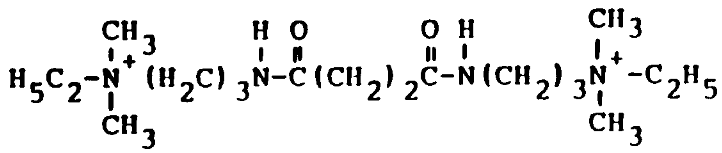 | $2BF_4^-$ |
| 6.3b | s.o. | 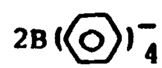 |

| Verbindung | Kation | Anion |
|---|---|---|
| 6.4a | 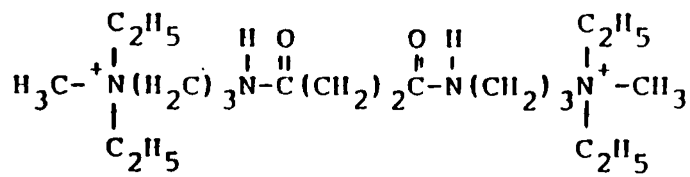 | $2BF_4^-$ |
| 6.4b | s.o. | 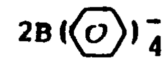 |
| 6.4c | s.o. | 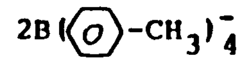 |
| 6.4d | s.o. | 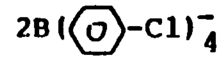 |
| 6.4e | s.o. | 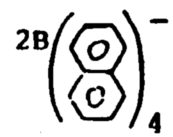 |
| 6.5a | 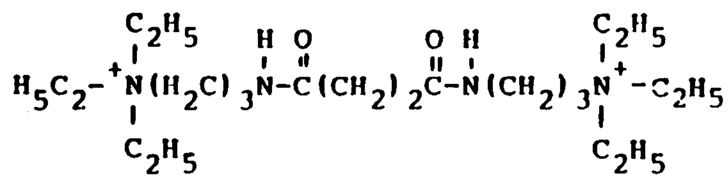 | $2BF_4^-$ |
| 6.5b | s.o. | 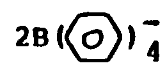 |

| Verbindung | Kation | Anion |
|---|---|---|
| 7.1a | 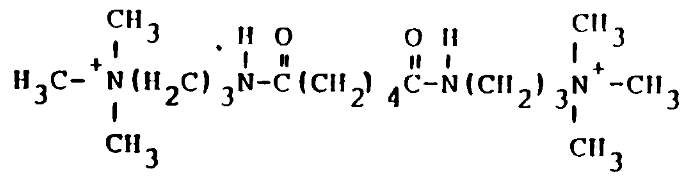 | $2BF_4^-$ |
| 7.1b | s.o. | 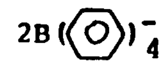 |
| 8.1a | 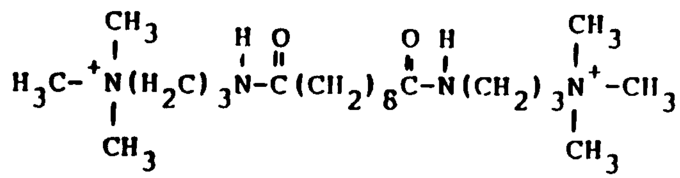 | $2BF_4^-$ |
| 8.1b | s.o. | 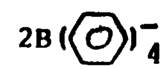 |
| 9.1a | 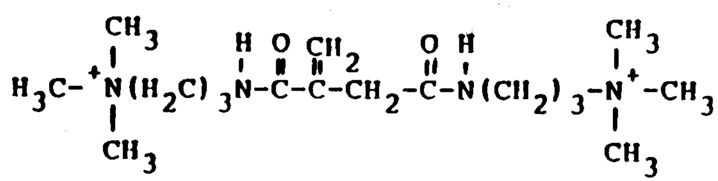 | $2BF_4^-$ |
| 9.1b | s.o. | 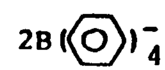 |

| Verbindung | Kation | Anion |
|---|---|---|
| 10.1a | 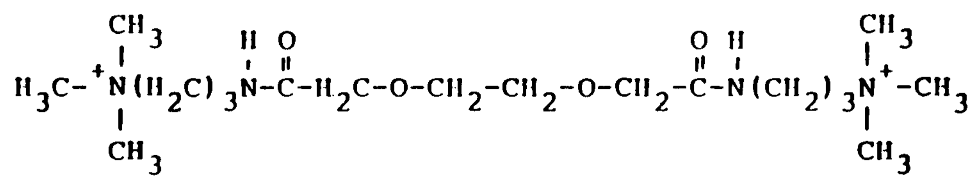 | $2BF_4^-$ |
| 10.1b | s.o. | 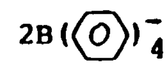 |
| 11.1a | 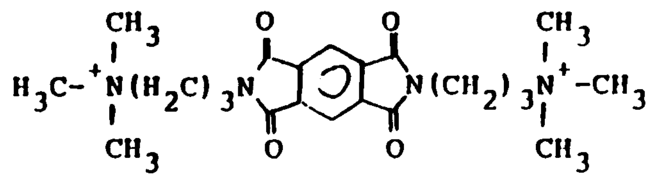 | $2BF_4^-$ |
| 11.1b | s.o. | 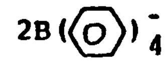 |
| 11.1c | s.o. | 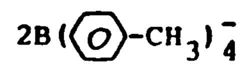 |
| 11.1d | s.o. | 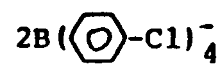 |
| 11.1e | s.o. | 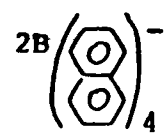 |

| Verbindung | Kation | Anion |
|---|---|---|
| 11.2a | 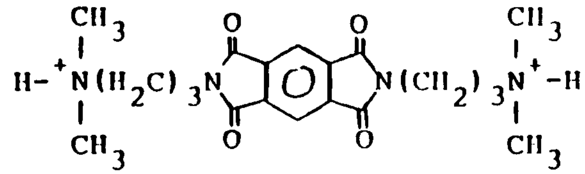 | $2BF_4^-$ |
| 11.2b | s.o. | 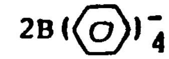 |
| 12.1a | 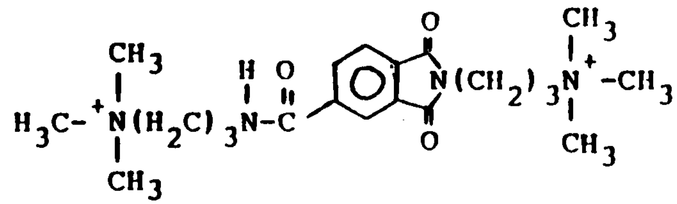 | $2BF_4^-$ |
| 12.1b | s.o. | 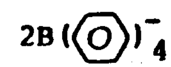 |
| 13.1a | 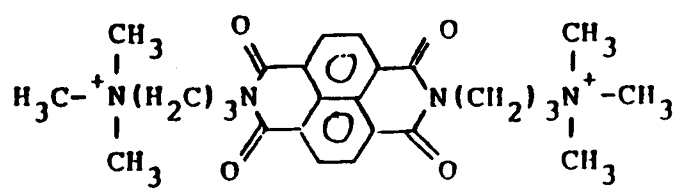 | $2BF_4^-$ |
| 13.1b | s.o. | 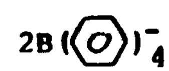 |
| 13.1c | s.o. | 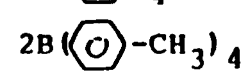 |

| Verbindung | Kation | Anion |
|---|---|---|
| 13.1d | 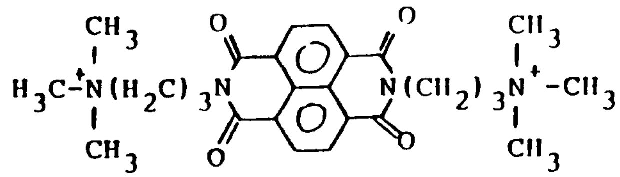 | 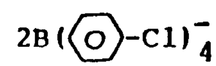 |
| 13.1c | s.o. | 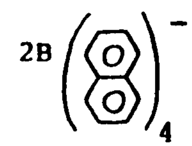 |
| 13.2a | 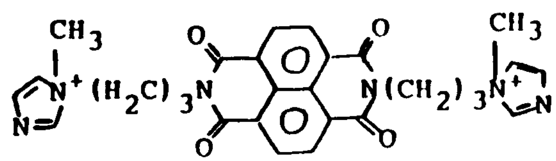 | $2BF_4^-$ |
| 13.2b | s.o. | 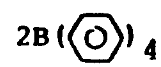 |
| 13.3a | 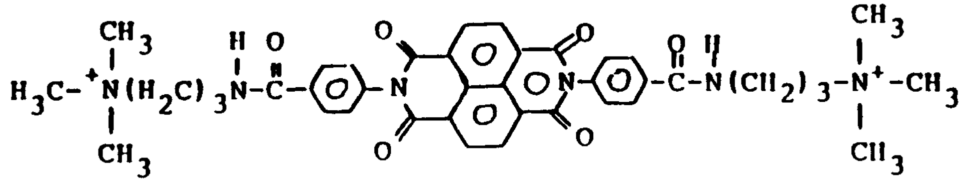 | $2BF_4^-$ |
| 13.3b | s.o. | 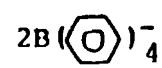 |

| Verbindung | Kation | Anion |
| --- | --- | --- |
| 14.1a | 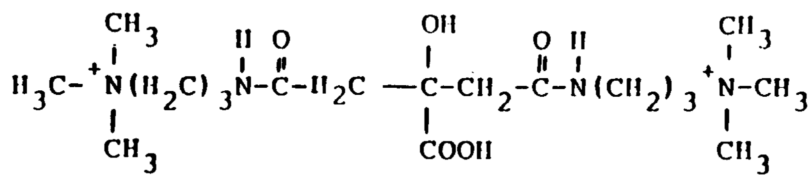 | $2BF_4^-$ |
| 14.1b | s.o. | 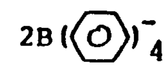 |
| 15.1a | 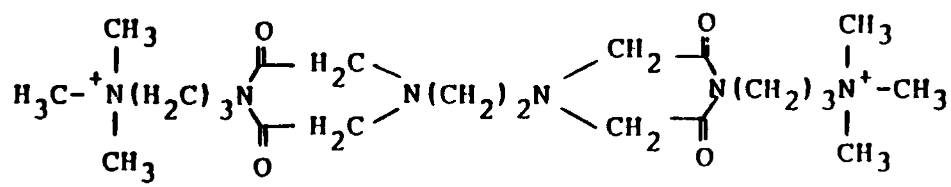 | $2BF_4^-$ |
| 15.1b | s.o. | 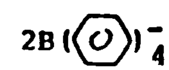 |
| 16.1a | 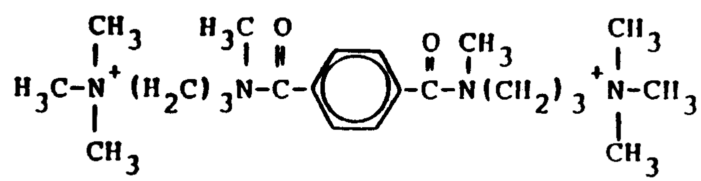 | $2BF_4^-$ |
| 16.1b | s.o. | 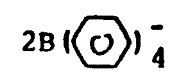 |

| Verbindung | Kation | Anion |
|---|---|---|
| 17.1a | 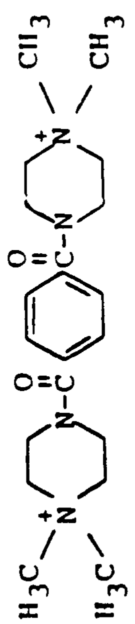 | $2BF_4^-$ |
| 17.1b | s.o. | 2B 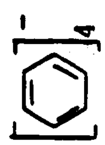 |

Dargestellt werden können die biskationischen Säureamid- und -imidderivate sowie deren Gemische und Mischkristalle mit gemischten Anionen und/oder Kationen der oben genannten allgemeinen Formeln (I) bis (III) durch Umsetzen der Säureamid- bzw. -imidderivate der allgemeinen Formeln (IV) bis (VI)

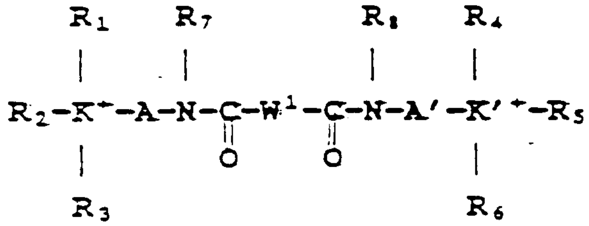

2 X⁻,

(IV)

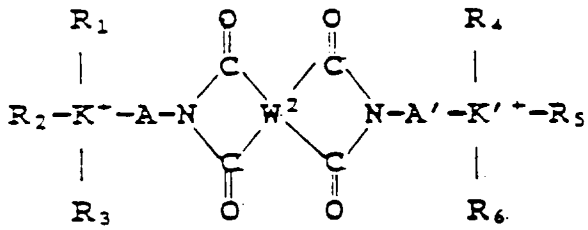

2 X⁻,

(V)

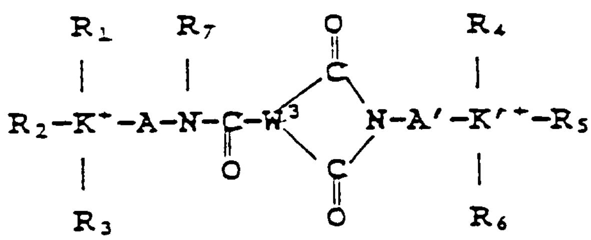

2 X⁻,

(VI)

wobei $R_1$ bis $R_8$, A und A', K und K' sowie $W^1$, $W^2$ und $W^3$ die oben genannten Bedeutungen haben und $X^-$ das stöchiometrische Äquivalent eines oder mehrerer Halogenanion(en), beispielsweise Chlor-, Brom- oder Jodanion(en), Acetat-, Methylsulfat-, Ethylsulfat-, Hydrogensulfat- oder Sulfatanion(en) bedeutet, mit einem Boratsalz in Wasser oder Gemischen aus Wasser und Säure, wie beipielsweise Essigsäure, oder Gemischen aus Wasser und organischem Lösungsmittel, wie beispielsweise Isopropanol, Isobutanol oder Methylisobutylketon, bei Temperaturen von etwa 10 °C bis etwa 90 °C, bevorzugt von etwa 20 °C bis etwa 40 °C. Die Verbindungen, Verbindungsgemische oder Mischkristalle der oben genannten allgemeinen Formeln (I) bis (III) fallen hierbei in guter Ausbeute und Reinheit an und können direkt aus dem Reaktionsmedium durch Abfiltrieren isoliert werden.

Die Herstellung der Verbindungen der allgemeinen Formeln (IV) bis (VI) geschieht in an sich bekannter Weise und ist in der Literatur ausführlich beschrieben (z. B. Houben-Weyl, "Methoden der Organischen Chemie", Georg Thieme Verlag, 1985, Band E 5, Teil 2, S. 924 - 1134 und a. a. Ort, 1958, Band 11/2, S. 591 - 630).

So erfolgt die Herstellung der Verbindungen (IV) z. B. durch Umsetzung der aliphatischen, cycloaliphatischen, araliphatischen oder iso- bzw. heterocyclischen aromatischen Dicarbonsäuren oder geeigneter

Dicarbonsäurederivate, wie z. B. deren Ester, Amide, Säurechloride oder Säureanhydride, mit Aminen oder Aminoverbindungen, die mindestens eine tertiäre und mindestens eine primäre oder sekundäre Aminogruppe enthalten, in einem inerten Reaktionsmedium oder in überschüssigem Amin als Reaktionsmedium und anschließender Bis-Protonierung mit einer anorganischen oder organischen Säure bzw. Bis-Quaternisierung mit einem geeigneten Quaternisierungsreagenz. Bevorzugt ist für die Herstellung der Verbindungen (IV) der Einsatz von Dicarbonsäuredihalogeniden oder von Dicarbonsäurediestern als Ausgangsprodukte. Besonders bevorzugt ist als Herstellungsverfahren die Aminolyse von Dicarbonsäurediestern, insbesondere der Dimethyl- oder Diethylester, mit Aminen bei erhöhter Temperatur unter Abdestillieren des gebildeten Alkohols.

Die Herstellung der Verbindungen (V) erfolgt z. B. durch Umsetzung der aliphatischen, cycloaliphatischen, araliphatischen oder iso- bzw. heterocyclischen aromatischen Tetracarbonsäuren oder geeigneter Derivate, wie z. B. deren Ester, Amide, Säurechloride oder Säureanhydride, insbesondere deren Mono- oder Dianhydride, mit Aminen oder Aminoverbindungen, die mindestens eine tertiäre und mindestens eine primäre Aminogruppe enthalten, und anschließender Bis-Protonierung mit einer anorganischen oder organischen Säure bzw. Bis-Quaternisierung mit einem geeigneten Quaternisierungsreagenz. Die Umsetzung kann sowohl säurekatalysiert in wäßrigem Medium als auch in aliphatischen oder aromatischen Carbonsäuren oder in Gemischen aus Wasser und derartigen Carbonsäuren erfolgen. Eine besonders geeignete Carbonsäure ist die Essigsäure. Die Herstellung der Verbindungen (V) kann aber auch,gegebenenfalls säurekatalysiert, in, gegebenenfalls substituierten, aliphatischen oder aromatischen Kohlenwasserstoffen bei erhöhter Temperatur unter Auskreisen des gebildeten Wassers erfolgen. Als besonders geeignete Kohlenwasserstoffe seien Toluol, Xylol, Chlorbenzol oder o-Dichlorbenzol genannt. Als Tetracarbonsäuren können prinzipell alle diimidbildenden Tetracarbonsäuren verwendet werden. Bevorzugt sind aromatische Tetracarbonsäuren, bei denen jeweils zwei Carbonsäuregruppen in ortho- oder peri-Stellung zueinander stehen.

Die Herstellung der Verbindungen (VI) erfolgt durch geeignete Kombination der für die Verbindungen (IV) und (V) beschriebenen Verfahren.

Für die Bis-Protonierung bzw. Bis-Quaternisierung kommen prinzipiell alle geeigneten anorganischen und organischen Säuren bzw. alle geeigneten Alkylierungsmittel in Betracht. Besonders geeignet als Säuren sind Salzsäure, Schwefelsäure und Essigsäure. Als Alkylierungsmittel sind Alkylhalogenide und Dialkylsulfate bevorzugt, insbesondere Methylchlorid, Methyljodid, Dimethyl- und Diethylsulfat. Als Reaktionsmedium für die Durchführung der Alkylierung kommen bevorzugt inerte Reaktionsmedien, wie beispielsweise Dimethylformamid oder aromatische Kohlenwasserstoffe in Betracht. Geeignet sind aber auch wasserfreie oder wasserhaltige Alkohole, wie beispielsweise Isobutanol oder das Isobutanol-Wasser-Azeotrop mit einem Wassergehalt von etwa 16 Gewichts-%. In einzelnen Fällen kann die Quaternisierung auch in wäßrigem Medium durchgeführt werden.

So werden beispielsweise die vorstehenden Verbindungen der Formeln 1.1a,b, 1.2a,b, 2.1.a,b, 3.1a,b, 4.1a,b, 6.1a,b, 6.2a,b, 11.1a,b, 11.2 a,b, 12.1a,b, 13.1a,b und 17.1a,b durch Umsetzen der nachstehenden Ausgangsverbindungen 1.1, 1.2, 2.1, 3.1, 4.1, 6.1, 6.2, 11.1, 11.2, 12.1, 13.1 und 17.1

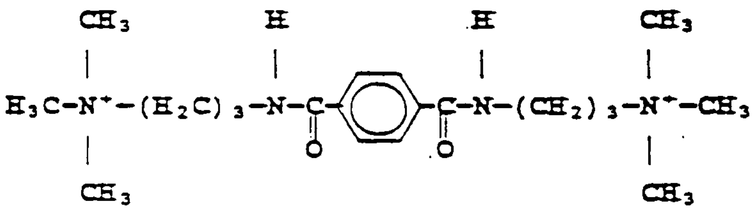 $(H_3COSO_3^-)_2$ 1.1

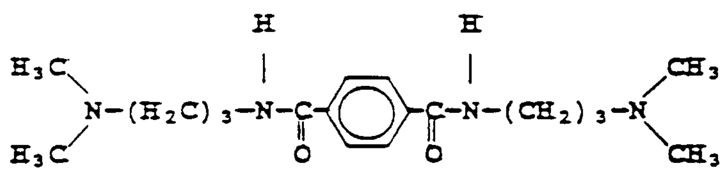 1.2

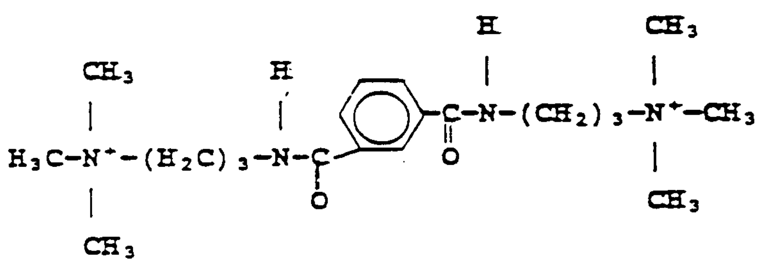 $(H_3COSO_3^-)_2$ 2.1

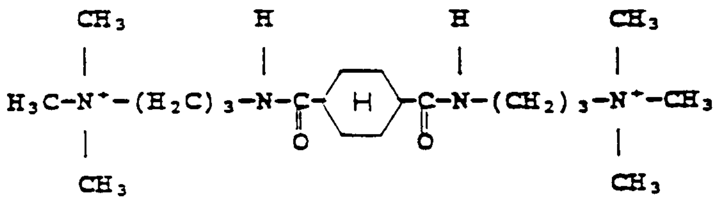

$(H_3COSO_3^-)_2$ 3.1

cis/trans

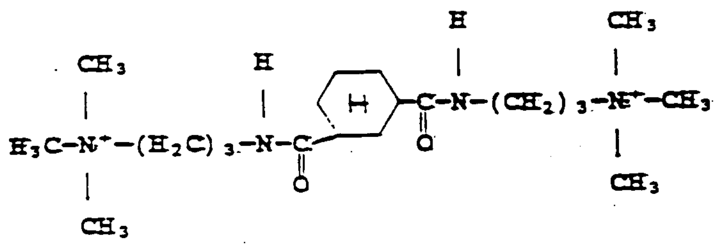

$(H_3COSO_3^-)_2$ 4.1

cis/trans

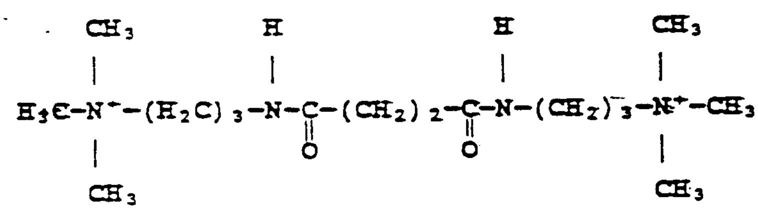

$(H_3COSO_3^-)_2$ 6.1

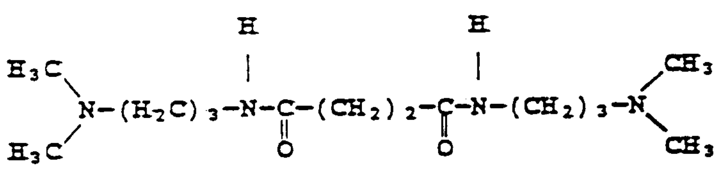

6.2

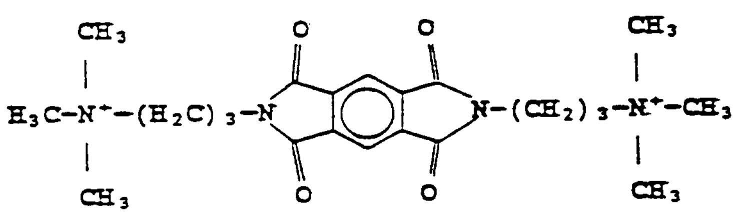

$(H_3COSO_3^-)_2$ 11.1

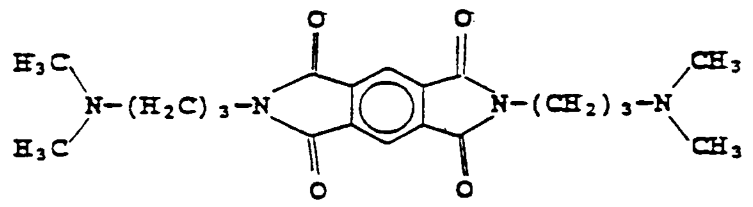

11.2

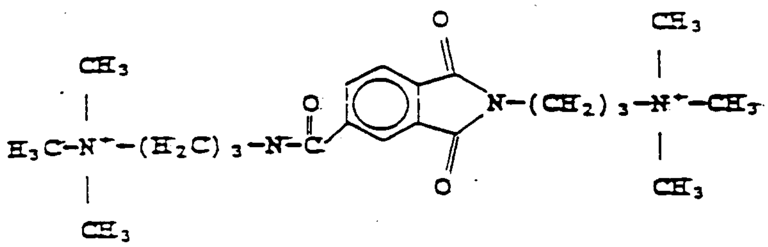

$(H_3COSO_3^-)_2$ 12.1

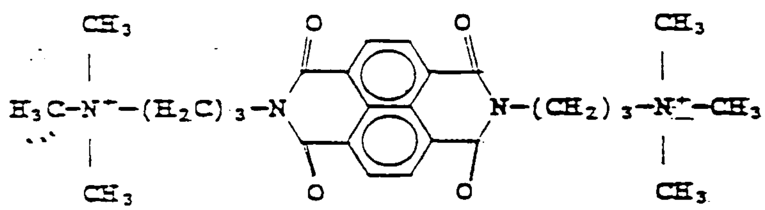

$(H_3COSO_3^-)_2$ 13.1

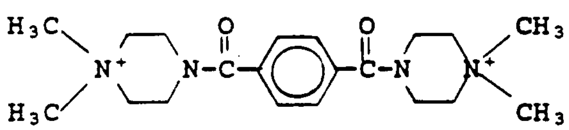

$(H_3COSO_3^-)_2$ 17.1

mit Natriumtetraphenylborat oder Natriumtetrafluoroborat dargestellt (siehe nachfolgende Herstellungsbeispiele 1 bis 20).

Die erfindungsgemäßen biskationischen Säureamid- und -imidderivate sind hervorragend geeignet für den Einsatz als farblose Ladungssteuermittel in Tonern und Entwicklern für elektrophotographische Aufzeichnungsverfahren sowie für den Einsatz als ladungsverbesserndes Mittel in Pulvern und Lacken zur Oberflächenbeschichtung, insbesondere in triboelektrisch bzw. elektrokinetisch versprühten Pulverlacken.

Die nachstehenden Herstellungsbeispiele dienen zur Erläuterung der Erfindung, ohne diese darauf zu beschränken.

Herstellungsbeispiel 1

149,2 g (0,73 Mol) Terephthalsäuredichlorid werden in 3,5 l wasserfreiem Toluol verrührt und anschließend unter Kühlung 180,0 g (1,76 Mol) 3-Dimethylamino-1-propylamin bei 20 bis 30 °C in 30 Minuten zugetropft. Man rührt 5 Stunden bei dieser Temperatur, erhitzt anschließend jeweils 1 bis 2 Stunden auf 50 bis 60 °C bzw. 70 bis 80 °C und erhitzt dann 4 Stunden zum Rückfluß. Das erhaltene Produkt wird bei 20 bis 30 °C abgesaugt, mit Toluol gewaschen und bei 100 °C im Vakuumschrank getrocknet. Man erhält 292,4 g (0,72 Mol) des Bisamides in Form des Bishydrochlorides. Das Produkt wird in 450 ml Wasser gelöst und bei 0 bis 5 °C innerhalb von 30 Minuten mit 180 g 33 %iger Natronlauge versetzt. Dabei fällt das Bisamid in grobkristalliner Form aus. Nach einstündigem Rühren bei 0 bis 5 °C wird das Produkt abgesaugt, mit 90 ml Eiswasser gewaschen und bei 100 °C im Vakuumschrank getrocknet.

Ausbeute: 221,2 g (90,7 % der Theorie) der Verbindung 1.2, weißes Pulver

| | |
|---|---|
| Molekulargewicht: | 334 |
| Schmelzpunkt : | 172 - 174 °C |
| $^1$H-NMR (in DMSO-$d_6$): | 1,65 (Quintett, 4 Methylen-H), 2,13 (Singulett, 12 Methyl-H), 2,28 (Triplett, 4 Methylen-H), 3,30 (Quartett, 4 Methylen-H), 7,90 (Singulett, 4 Phenylen-H), 8,63 (Triplett, 2 Amid-H) ppm. |

Herstellungsbeispiel 2

66,8 g (0,2 Mol) der Verbindung 1.2 werden in 1,6 l Toluol verrührt und bei 20 bis 30 °C innerhalb von 10 Minuten mit 100,8 g (0,8 Mol) Dimethylsulfat versetzt. Man rührt 1 Stunde bei 20 bis 30 °C und erhitzt dann 5 Stunden am Rückfluß. Nach Abkühlung auf 20 bis 30 °C wird das Produkt abgesaugt, mit Toluol gewaschen und bei 100 °C im Vakuumschrank getrocknet.

| | |
|---|---|
| Ausbeute: | 112,7 g (96,2 % der Theorie) der Verbindung 1.1, weißes Pulver |
| Molekulargewicht: | 586 |
| Schmelzpunkt: | 180 °C |
| $^1$H-NMR (in $D_2O$): | 2,18 (Multiplett, 4 Methylen-H), 3,20 (Singulett, 18 Methyl-H), 3,50 (Multiplett, 8 Methylen-H), 3,75 (Singulett, 6 Methyl-H), 7,88 (Singulett, 4 Phenylen-H) ppm. |

Herstellungsbeispiel 3

Zu 100 ml einer wäßrigen Lösung von 10,0 g (17 mMol) Verbindung 1.1 wird bei Raumtemperatur unter Rühren eine Lösung von 13,7 g (40 mMol) Natriumtetraphenylborat in 50 ml Wasser zugetropft. Dabei fällt Verbindung 1.1b als weißer Niederschlag aus. Der Niederschlag wird abgesaugt, mit Wasser gewaschen und im Umluftschrank bei 60 °C getrocknet.

| | |
|---|---|
| Ausbeute: | 17,0 g (99,8 % der Theorie) der Verbindung 1.1 b, weißes Pulver |
| Molekulargewicht: | 1002 |
| Schmelzpunkt: | 255 °C |
| $^1$H-NMR (in DMSO-$d_6$): | 1,98 (Multiplett, 4 Methylen-H), 3,03 (Singulett, 18 Methyl-H), 3,38 (Multiplett, 8 Methylen-H), 6,96 (Multiplett, 40 Phenyl-H), 7,95 (Singulett, 4 Phenylen-H), 8,64 (Triplett, 2 Amid-H) ppm. |

Herstellungsbeispiel 4

Es wird wie in Herstellungsbeipiel 3 verfahren, mit dem Unterschied, daß anstelle von Natriumtetraphenylborat-Lösung eine Lösung von 4,4 g (40 mMol) Natriumtetrafluoroborat in 50 ml Wasser verwendet und die Reaktionslösung auf 30 ml eingeengt und auf 2 °C abgekühlt wird.

| | |
|---|---|
| Ausbeute: | 8,9 g (97,3 % der Theorie) der Verbindung 1.1a, weißes Pulver |
| Molekulargewicht: | 538 |
| Schmelzpunkt: | 228 °C |
| $^1$H-NMR (in DMSO-$d_6$): | 1,99 (Multiplett, 4 Methylen-H), 3,05 (Singulett, 18 Methyl-H), 3,38 (Multiplett, 8 Methylen-H), 7,93 (Singulett, 4 Phenylen-H), 8,62 (Triplett, 2 Amid-H) ppm. |

Herstellungsbeispiel 5

5,0 g (15 mMol) der Verbindung 1.2 werden in 50 ml Wasser suspendiert und bis zum Erreichen von pH 7 mit 2 N Essigsäure versetzt, wobei das Amin in Lösung geht. Anschließend wird eine Lösung von 13,7 g (40 mMol) Natriumtetraphenylborat in 50 ml Wasser zugetropft, wobei das Produkt als dicker weißer Niederschlag ausfällt. Die Reaktionsmischung wird 30 min. bei Raumtemperatur gerührt, der Niederschlag abgesaugt, mit Wasser gewaschen und bei 60 °C im Umluftschrank getrocknet.

| | |
|---|---|
| Ausbeute: | 14,5 g (99,2 % der Theorie) der Verbindung 1.2 b, weißes Pulver |
| Molekulargewicht: | 974 |
| Schmelzpunkt: | 197 °C |
| $^1$H-NMR (in DMSO-$d_6$): | 1,85 (Multiplett, 4 Methylen-H), 2,71 (Singulett, 12 Methyl-H), 3,00 (Multiplett, 4 Methylen-H), 3,35 (Muliplett, 4 Methylen-H) 6,96 (Multiplett, 40 Phenyl-H), 7,93 (Singulett, 4 Phenylen-H), 8,63 (Triplett, 2 Amid-H) ppm. |

Herstellungsbeispiel 6

109,5 g (0,75 Mol) Bernsteinsäuredimethylester werden in 459 g (4,5 Mol) 3-Dimethylamino-1-propylamin gelöst. Dann erhitzt man 10 Stunden unter Rückfluß. Da die Siedetemperatur durch die rasch einsetzende Methanolabspaltung erheblich absinkt, sorgt man durch gelegentliches Abdestillieren eines Methanol-Amin-Gemisches dafür, daß die Temperatur in der Gasphase oberhalb von 125 °C bleibt. Gegen Ende der Reaktionsdauer liegt die Temperatur in der Gasphase oberhalb von 130 °C. Im Verlauf der Reaktion werden etwa 200 g Methanol-Amin-Gemisch abdestilliert. Danach wird auf 20 bis 30 °C abgekühlt und das auskristallisierte Reaktionsprodukt abgesaugt. Aus dem Filtrat läßt sich durch dreifache Verdünnung mit Benzin weiteres Produkt ausfällen. Das Produkt wird mit Benzin aminfrei gewaschen und bei 100 °C im Vakuumschrank getrocknet.

| | |
|---|---|
| Ausbeute: | 188,0 g (87,6 % der Theorie) der Verbindung 6.2, weißes Pulver |
| Molekulargewicht: | 286 |
| Schmelzpunkt: | 126 - 128 °C |
| $^1$H-NMR (in DMSO-$d_6$): | 1,48 (Quintett, 4 Methylen-H), 2,08 (Singulett, 12 Methyl-H), 2,20 (Triplett, 4 Methylen-H), 2,25 (Singulett, 4 Methylen-H), 3,05 (Quartett, 4 Methylen-H), 7,78 (Triplett, 2 Amid-H) ppm. |

Herstellungsbeispiel 7

85,8 g (0,3 Mol) 6.2 werden in 610 ml wasserfreies Dimethylformamid eingetragen. Bei Raumtemperatur entsteht rasch eine klare Lösung. Dann läßt man bei 30 bis 40 °C in etwa 15 Minuten 189 g (1,5 Mol) Dimethylsulfat zutropfen. Nach kurzer Zeit entsteht ein dicker Kristallbrei, der bei Erwärmung auf 60 °C in eine gut rührbare Suspension übergeht. Es wird 5 Stunden bei 60 bis 70 °C nachgerührt und das Produkt nach Abkühlung auf 0 bis 5 °C abgesaugt. Es wird gründlich mit Toluol gewaschen und im Vakuumschrank bei 100 °C getrocknet.

| | |
|---|---|
| Ausbeute: | 151,0 g (93,6 % der Theorie) der Verbindung 6.1, weißes Pulver |
| Molekulargewicht: | 538 |
| Schmelzpunkt: | 152 °C |
| $^1$H-NMR (in DMSO-$d_6$): | 1,85 (Multiplett, 4 Methylen-H), 2,33 (Singulett, 4 Methylen-H), 3,05 (Singulett, 18 Methyl-H), 3,20 (Multiplett, 8 Methylen-H), 3,40 (Singulett, 6 Methyl-H), 7,95 (Triplett, 2 Amid-H) ppm. |

Herstellungsbeispiel 8

Es wird wie in Herstellungsbeispiel 3 verfahren, mit dem Unterschied, daß anstelle der Verbindung 1.1 10,0 g (18,5 mMol) der Verbindung 6.1 verwendet wird.

| | |
|---|---|
| Ausbeute: | 17,3 g (98,0 % der Theorie) der Verbindung 6.1 b, weißes Pulver |
| Molekulargewicht: | 954 |
| Schmelzpunkt: | 245 °C |
| $^1$H-NMR (in DMSO-$d_6$): | 1,81 (Multiplett, 4 Methylen-H), 2,37 (Singulett, 4 Methylen-H), 3,02 - (Singulett, 18 Methyl-H), 3,12 (Quartett, 4 Methylen-H), 3,24 (Multiplett, 4 Methylen-H), 6,97 (Multiplett, 40 Phenyl-H), 7,90 (Triplett, 2 Amid-H) ppm. |

Herstellungsbeispiel 9

Es wird wie in Herstellungsbeispiel 5 verfahren, mit dem Unterschied, daß anstelle der Verbindung 1.2 5,0 g (17,5 mMol) der Verbindung 6.2 verwendet wird.

| | |
|---|---|
| Ausbeute: | 15,7 g (96,9 % der Theorie) der Verbindung 6.2 b, weißes Pulver |
| Molekulargewicht: | 926 |
| Schmelzpunkt: | 183 °C |
| $^1$H-NMR (in DMSO-$d_6$): | 1,72 (Multiplett, 4 Methylen-H), 2,38 (Singulett, 4 Methylen-H), 2,68 (Singulett, 12 Methyl-H), 2,95 (Multiplett, 4 Methylen-H), 3,12 (Quartett, 4 Methylen-H), 7,01 (Multiplett, 40 Phenyl-H), 7,92 (Triplett, 2 Amid-H) ppm. |

Herstellungsbeispiel 10

218,0 g (1,0 Mol) Pyromellitsäuredianhydrid werden in 1,2 l Eisessig verrührt und bei 40 bis 50 °C 306 g (3,0 Mol) 3-Dimethylamino-1-propylamin unter Kühlung zugetropft. Anschließend erhitzt man 3 Stunden zum Rückfluß, setzt 920 ml o-Dichlorbenzol zu und destilliert die Hauptmenge des Eisessigs ab. Dann erhitzt man unter Überleiten eines Stickstoffstromes und Abdestillieren des restlichen Eisessigs bis die Siedetemperatur von o-Dichlorbenzol erreicht ist und erhitzt 6 Stunden weiter am Rückfluß. Nach Abkühlung auf 20 bis 30 °C wird das ausgefallene Produkt abgesaugt, mit Benzin gewaschen und bei 100 °C im Vakuumschrank getrocknet.

| | |
|---|---|
| Ausbeute: | 271,0 g (70,2 % der Theorie) der Verbindung 11.2, weißes Pulver |
| Molekulargewicht: | 386 |
| Schmelzpunkt: | 186 - 188 °C |
| $^1$H-NMR (in DMSO-$d_6$): | 1,75 (Quintett, 4 Methylen-H), 2,08 (Singulett, 12 Methyl-H), 2,25 (Triplett, 4 Methylen-H), 3,68 (Triplett, 4 Methylen-H), 8,15 (Singulett, 2 Phenylen-H) ppm. |

Herstellungsbeispiel 11

77,2 g (0,2 Mol) der Verbindung 11.2 werden in 600 ml Dimethylformamid verrührt und bei 30 bis 40 °C 126,0 g (1,0 Mol) Dimethylsulfat unter Kühlung innerhalb von 20 Minuten zugetropft. Man erhitzt 5 Stunden auf 130 bis 135 °C und saugt das Produkt nach Abkühlung auf 20 bis 30 °C ab. Das Produkt wird mit Toluol gewaschen und bei 100 °C im Vakuumschrank getrocknet.

| | |
|---|---|
| Ausbeute: | 121,9 g (95,5 % der Theorie) der Verbindung 11.1, weißes Pulver |
| Molekulargewicht: | 638 |
| Schmelzpunkt: | 299 °C |
| $^1$H-NMR (in $D_2O$): | 2,23 (Multiplett, 4 Methylen-H), 3,15 (Singulett, 18 Methyl-H), 3,48 (Multiplett, 4 Methylen-H), 3,73 (Singulett, 6 Methyl-H),3,88 (Triplett, 4 Methylen-H), 8,33 (Singulett, 2 Phenylen-H) ppm. |

Herstellungsbeispiel 12

Es wird wie in Herstellungsbeispiel 4 verfahren, mit dem Unterschied, daß anstelle der Verbindung 1.1 10,0 g (16 mMol) Verbindung 11.1 verwendet und weder eingeengt noch abgekühlt wird.

| | |
|---|---|
| Ausbeute: | 8,4 g (89,0 % der Theorie) der Verbindung 11.1a, weißes Pulver |
| Molekulargewicht: | 590 |
| Schmelzpunkt: | > 300 °C |
| $^1$H-NMR (in DMSO-$d_6$): | 2,12 (Multiplett, 4 Methylen-H), 3,05 (Singulett, 18 Methyl-H), 3,35 (Multiplett, 4 Methylen-H), 3,71 (Triplett, 4 Methylen-H), 8,26 (Singulett, 2 Phenylen-H) ppm. |

Herstellungsbeispiel 13

Es wird wie in Herstellungsbeispiel 12 verfahren, mit dem Unterschied, daß anstelle von Natriumtetrafluoroborat-Lösung eine Losung von 13,7 g (40 mMol) Natriumtetraphenylborat in 50 ml Wasser verwendet wird.

| | |
|---|---|
| Ausbeute: | 16,8 g (99,6 % der Theorie) der Verbindung 11.1 b, hellgelbes Pulver |
| Molekulargewicht: | 1054 |
| Schmelzpunkt: | 295 °C |
| $^1$H-NMR (in DMSO-$d_6$): | 2,08 (Multiplett, 4 Methylen-H), 2,97 (Singulett, 18 Methyl-H), 3,36 (Multiplett, 4 Methylen-H), 3,70 (Triplett, 4 Methylen-H), 6,98 (Multiplett, 40 Phenyl-H) 8,28 (Singulett, 2 Phenylen-H) ppm. |

Herstellungsbeispiel 14

71,5 g (0,25 Mol) Naphthalin-1,4,5,8-tetracarbonsäure-1,8-monoanhydrid werden in 500 ml Eisessig verrührt und 93,8 g (0,75 Mol) N-(3-aminopropyl)-imidazol bei 40 bis 50 °C unter leichter Kühlung zugetropft. Anschließend erhitzt man 6 Stunden am Rückfluß. Die entstandene Losung des Reaktionsproduktes gießt man in 2,5 l Wasser und tropft dann bei 20 bis 30 °C unter Kühlung 1,01 kg 33 %ige

Natronlauge zu, wobei das Produkt ausfällt. Es wird abgesaugt, gründlich mit Wasser gewaschen und bei 100 °C im Vakuumschrank getrocknet.

| | |
|---|---|
| Ausbeute: | 116,4 g (96,6 % der Theorie) der Verbindung 13.4, weißes Pulver |
| Molekulargewicht: | 482 |
| Schmelzpunkt: | 260 - 263 °C |

Herstellungsbeispiel 15

96,4 g (0,2 Mol) der Verbindung 13.4 werden in 600 ml Dimethylformamid verrührt und 189,0 g (1,5 Mol) Dimethylsulfat innerhalb von 10 Minuten bei 30 bis 40 °C unter leichter Kühlung zugetropft. Anschließend rührt man 5 Stunden bei 130 bis 135 °C. Nach Abkühlung auf 20 bis 30 °C wird das Produkt abgesaugt, mit 100 ml Dimethylformamid, dann mit Toluol gewaschen und bei 100 °C im Vakuumschrank getrocknet.

| | |
|---|---|
| Ausbeute: | 141,8 g (96,6 % der Theorie) der Verbindung 13.2, hellgelbes Pulver |
| Molekulargewicht: | 734 |
| Schmelzpunkt: | 261 °C |
| $^1$H-NMR (in $D_2O$): | 2,35 (Quintett, 4 Methylen-H), 3,68 (Singulett, 6 Methyl-H), 3,95 (Singulett, 6 Methyl-H), 4,10 (Triplett, 4 Methylen-H), 4,39 (Triplett, 4 Methylen-H), 7,60 (Multiplett, 4 Imidazoyl-H), 8,40 (Singulett, 4 Naphthylen-H), 8,86 (Singulett, 2 Imidazoyl-H) ppm. |

Herstellungsbeispiel 16

Es wird wie in Herstellungsbeispiel 4 verfahren, mit dem Unterschied, daß anstelle von Verbindung 1.1 10,0 g (15 mMol) der Verbindung 13.1 verwendet und weder eingeengt noch abgekühlt wird.

| | |
|---|---|
| Ausbeute: | 9,3 g (96,9 % der Theorie der Verbindung 13.1 a, hellgelbes Pulver |
| Molekulargewicht: | 640 |
| Schmelzpunkt: | > 300 °C (Zersetzung) |
| $^1$H-NMR (in DMSO-$d_6$): | 2,20 (Multiplett, 4 Methylen-H), 3,05 (Singulett, 18 Methyl-H), 3,46 (Multiplett, 4 Methylen-H), 4,19 (Triplett, 4 Methylen-H), 8,68 (Singulett, 4 Naphthylen-H) ppm. |

Herstellungsbeispiel 17

Es wird wie in Herstellungsbeispiel 16 verfahren, mit dem Unterschied, daß anstelle von Natriumtetrafluoroborat-Lösung eine Losung von 13,7 g (40 mMol) Natriumtetraphenylborat in 50 ml Wasser verwendet wird.

| | |
|---|---|
| Ausbeute: | 14,0 g (84,5 % der Theorie) der Verbindung 13.1 b, gelbes Pulver |
| Molekulargewicht: | 1104 |
| Schmelzpunkt: | 287 °C (Zersetzung) |
| $^1$H-NMR (in DMSO-$d_6$): | 2,13 (Multiplett, 4 Methylen-H), 3,00 (Singulett, 18 Methyl-H), 3,45 (Multiplett, 4 Methylen-H), 4,16 (Triplett, 4 Methylen-H), 6,90 (Multiplett, 40 Phenyl-H), 8,71 (Singulett, 4 Naphthylen-H) ppm. |

Herstellungsbeispiel 18

Es wird wie in Herstellungsbeispiel 3 verfahren, mit dem Unterschied, daß anstelle der Verbindung 1.1 10,0 g (17 mMol) der Verbindung 3.1 verwendet wird.

| | |
|---|---|
| Ausbeute: | 7,8 g (45,5 % der Theorie) der Verbindung 3.1b, weißes Pulver |
| Molekulargewicht: | 1008 |
| Schmelzpunkt: | 255 °C |
| $^1$H-NMR (in DMSO-$d_6$): | 1,35 (Multiplett, 4 Methylen-H), 1,79 (Multiplett, 8 Cyclohexylen-H), 2,08 (Multiplett, 2 Cycohexylen-H), 3,01 (Singulett, 18 Methyl-H), 3,09 (Multiplett, 4 Methylen-H), 3,25 (Multiplett, 4 Methylen-H), 7,00 (Multiplett, 40 Phenyl-H), 7,81 (Triplett, 2 Amid-H) ppm. |

Herstellungsbeispiel 19

101,5 g (0,5 Mol) Terephthalsäuredichlorid werden in 2,3 l Toluol gelöst und unter Kühlung bei 20 bis 30 °C 120 g (1,2 Mol) N-Methylpiperazin zugetropft. Man rührt 1 Stunde bei 20 bis 30 °C, heizt anschließend über 2 Stunden bis zum Rückfluß hoch und kocht 4 Stunden am Rückfluß. Nach Abkühlen auf Raumtemperatur wird das Produkt abgesaugt, mit Toluol gewaschen und getrocknet. Das trockene Produkt wird in 400 ml Wasser gelöst, mit Aktivkohle und Kieselgur geklärt und das Bisamid durch Zugabe von 33 %iger NaOH bei 0 bis 5 °C ausgefällt. Das Bisamid wird abgesaugt, mit Wasser gewaschen und bei 100 °C im Vakuum getrocknet. 66 g (0,2 Mol) des trockenen Bisamids werden in 640 ml Dimethylformamid gelöst und 76 ml (0,8 Mol) Dimethylsulfat werden bei Raumtemperatur unter leichter Kühlung über 15 Minuten zugetropft. Anschließend wird 5 Stunden auf 60 bis 70 °C erwärmt und dann bei 0 bis 5 °C das Produkt abgesaugt, mit Toluol gewaschen und bei 100 °C im Vakuum getrocknet.

| | |
|---|---|
| Ausbeute: | 109 g (quantitative Ausbeute) der Verbindung 17.1, weißes Pulver |
| Molekulargewicht: | 582 |
| Schmelzpunkt: | > 300 °C |
| $^1$H-NMR (in $D_2O$): | 3,28 (Singulett, 12 Methyl-H), 3,53 (Multiplett, 8 H Piperazino-H), 3,73 (Singulett, Methyl-H des Methylsulfat-Anions, größtenteils zum Hydrogensulfat hydrolysiert), 3,88 (Multiplett, 4 Piperazino-H), 4,13 (Multiplett, 4 Piperazion-H), 7,60 (Singulett, 4 Phenylen-H) ppm. |

Herstellungsbeispiel 20

5,0 g (9 mMol) der Verbindung 17.1 werden bei Raumtemperatur in 20 ml Wasser gelöst. Anschließend werden 2,2 g (20 mMol) Natriumtetrafluoroborat in 25 ml Wasser langsam zugegeben, wobei die Reaktionsmischung durch ausfallende Kristalle sehr dick wird. Es wird auf 250 ml mit Wasser verdünnt, und die farblosen Kristalle dann abgenutscht. Nach waschen mit Wasser wird das Produkt bei 100 °C im Vakuumschrank getrocknet.

| | |
|---|---|
| Ausbeute: | 3,8 g (79,1 % der Theorie) der Verbindung 17.1a, weiße Kristalle |
| Molekulargewicht: | 534 |
| Schmelzpunkt: | > 300 °C |
| $^1$H-NMR (in DMSO-$d_6$): | 3,20 (Singulett, 12 Methyl-H), 3,48 (Singulett, 8 Piperazino-H), 3,83 (Singulett, 8 Piperazino-H), 7,56 (Singulett, 4 Phenylen-H) ppm. |

Herstellungsbeispiel 21

Es wird wie in Herstellungsbeispiel 20 verfahren, mit dem Unterschied, daß anstelle von Natriumtetrafluoroborat 7,0 g (20 mMol) Natriumtetraphenylborat, gelöst in 20 ml Wasser, verwendet werden.

| | |
|---|---|
| Ausbeute: | 7,6 g (84,6 % der Theorie) der Verbindung 17.1b, weißes Pulver |
| Molekulargewicht: | 998 |
| Schmelzpunkt: | 292 °C (Zersetzung) |
| $^1$H-NMR (in DMSO-$d_6$): | 3,19 (Singulett, 12 Methyl-H), 3,46 (Singulett, 8 Piperazino-H), 3,82 (Singulett, 8 Piperazino-H), 7,03 (Multiplett, 40 Phenyl-H), 7,55 (Singulett, 4 Phenylen-H) ppm. |

## Patentansprüche

1. Biskationische Säureamid- und -imdiderivate der allgemeinen Formeln (I) bis (III)

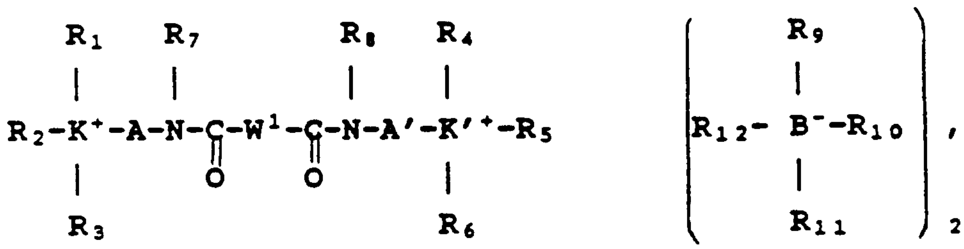

(I)

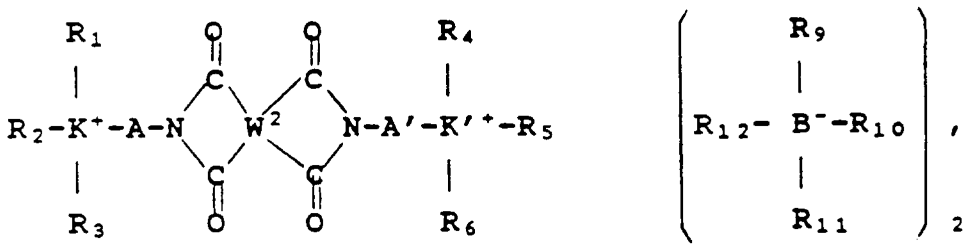

(II)

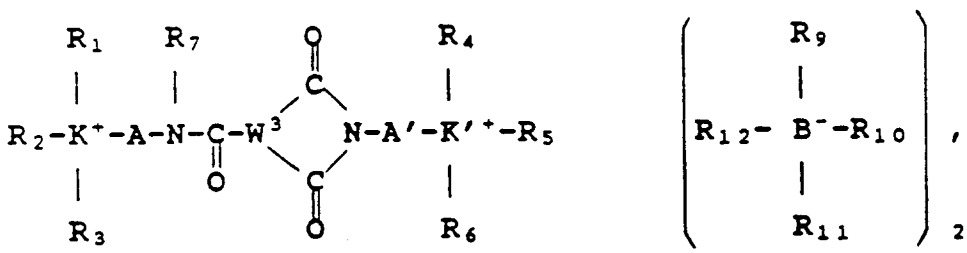

(III)

worin

$R_1$ bis $R_8$ unabhängig voneinander jeweils ein Wasserstoffatom oder jeweils ein Rest auf Basis eines Kohlenwasserstoffes, der durch Heteroatome unterbrochen sein kann, bedeuten,

wobei die Reste $R_1$ und $R_2$ bzw. $R_4$ und $R_5$ sich unabhängig voneinander unter Einbeziehung von K bzw. K' zu einem Ringsystem zusammenschließen können und für den Fall, daß $R_1$ oder $R_2$ bzw. $R_4$ oder $R_5$ in diesem Zusammenhang eine Doppelbindung zu K oder K' ausbilden,dann $R_3$ bzw. $R_6$ gegenstandslos sind, und/oder einer der Reste $R_1$, $R_2$ oder $R_3$ sich mit $R_7$ bzw. einer der Reste $R_4$, $R_5$ oder $R_6$ sich mit $R_8$ zu einer aliphatischen Brücke aus 2 bis 5 Kohlenstoffatomen zusammenschließen kann,

und worin A und A' sowie $W^1$ als zweiwertiges, $W^2$ als vierwertiges und $W^3$ als dreiwertiges Brückenglied unabhängig voneinander jeweils ein Brückenglied auf Basis eines Kohlenwasserstoffes sind, das durch Heteroatome unterbrochen sein kann, wobei $W^1$ auch eine direkte Bindung darstellen kann, und K und K' unabhängig voneinander jeweils ein Stickstoff-, Phosphor-, Arsen- oder Antimonatom darstellen und die Reste $R_9$ bis $R_{12}$ unabhängig voneinander aliphatische, cycloaliphatische, iso- oder heterocyclische, aromatische oder araliphatische Reste, wobei die genannten aliphatischen, cycloaliphatischen, aromatischen und araliphatischen Reste durch Alkyl($C_1$-$C_4$)-, Alkoxy($C_1$-$C_4$)-, Arylreste oder Halogenatome substituiert sein können, oder Fluoratome bedeuten sowie Gemische dieser Verbindungen und Mischkristalle mit gemischten Anionen und/oder Kationen.

2. Biskationische Säureamid- und -imidderivate gemäß Anspruch 1, dadurch gekennzeichnet, daß in den in Anspruch 1 genannten Formeln (I) bis (III) K und K' jeweils ein Stickstoffatom darstellen.

3. Biskationische Säureamid- und -imidderivate nach mindestens einem der Ansprüche 1 bis 2, dadurch gekennzeichnet, daß in den in Anspruch 1 genannten Formeln (I) bis (III) $R_1$ bis $R_8$ unabhängig voneinander jeweils ein Wasserstoffatom, eine geradkettige oder verzweigte, gesättigte oder ungesättigte Alkylgruppe mit 1 bis 30 Kohlenstoffatomen, eine Polyoxalkylengruppe der allgemeinen Formel -[Alkylen($C_1$-$C_5$)-O]$_n$-R, worin R ein Wasserstoffatom, eine Alkyl($C_1$-$C_4$)gruppe, eine Acylgruppe und n eine Zahl von 1 bis 10 bedeuten, einen ein- oder mehrkernigen cycloaliphatischen Rest mit 5 bis 12 Kohlenstoffatomen, einen ein- oder mehrkernigen aromatischen oder araliphatischen Rest darstellen, wobei solche aliphatischen, cycloaliphatischen, araliphatischen und aromatischen Reste durch Carbon- oder Sulfonsäuregruppen, deren Salze bzw. Amide und Ester, Alkyl($C_1$-$C_4$)-, Hydroxy-, Alkoxy($C_1$-$C_4$)gruppen, primäre, sekundäre oder tertiäre Aminogruppen sowie durch Fluor-, Chlor- oder Bromatome, die aliphatischen Reste bevorzugt durch Fluoratome, substituiert sein können und wobei die genannten aliphatischen Reste sowie die cycloaliphatischen, araliphatischen oder aromatischen Ringsysteme ein oder mehrere Heteroatome enthalten können und $R_1$ und $R_2$ bzw. $R_4$ und $R_5$ sich unabhängig voneinander unter Einbeziehung von K bzw. K' zu einem gesättigten oder ungesättigten, aromatischen oder nichtaromatischen 5- bis 7-gliedrigen Ringsystem zusammenschließen können, das weitere Heteroatome enthalten sowie substituiert und/oder durch Ankondensation von oder Verbrückung zu weiteren Ringsystemen modifiziert sein kann, und wobei für den Fall, daß $R_1$ oder $R_2$ bzw. $R_4$ oder $R_5$ in diesem Zusammenhang eine Doppelbindung zu K bzw. K' ausbilden dann $R_3$ oder $R_6$ gegenstandslos sind.

4. Biskationische Säureamid- und -imidderivate nach mindestens einem der Ansprüche 1 bis 3, dadurch gekennzeichnet, daß in den in Anspruch 1 genannten Formeln (I) bis (III) A und A' unabhängig voneinander jeweils ein geradkettiges oder verzweigtes, gesättigtes oder ungesättigtes aliphatisches Brückenglied mit 1 bis 30 Kohlenstoffatomen, ein ein- oder mehrkerniges cycloaliphatisches Brückenglied, ein ein- oder mehrkerniges aromatisches oder araliphatisches Brükkenglied darstellen, wobei solche aliphatischen, cycloaliphatischen, araliphatischen und aromatischen Brückenglieder durch Carbon- oder Sulfonsäuregruppen, deren Salze bzw. Amide und Ester, Alkyl($C_1$-$C_4$)-, Hydroxy-, Alkoxy($C_1$-$C_4$)gruppen, primäre, sekundäre oder tertiäre Aminogruppen sowie durch Fluor-, Chlor- oder Bromatome, die aliphatischen Brückenglieder bevorzugt durch Fluoratome, substituiert sein können, und die genannten aliphatischen, cycloaliphatischen, araliphatischen oder aromatischen Brückenglieder ein oder mehrere Stickstoff- und/oder Sauerstoff- und/oder Schwefelatome enthalten können.

5. Biskationische Säureamid- und -imidderivate nach mindestens einem der Ansprüche 1 bis 4, dadurch gekennzeichnet, daß in den in Anspruch 1 genannten Formeln (I) bis (III) $W^1$ als zweiwertiges Brückenglied ein geradkettiges oder verzweigtes, gesättigtes oder ungesättigtes aliphatisches Zwischenglied mit 1 bis 30 Kohlenstoffatomen, ein Polyoxalkylenglied der allgemeinen Formel -[$CH_2$-O-(Alkylen($C_1$-$C_5$)-O]$_m$-$CH_2$-, wobei m eine Zahl von 0 bis 10 bedeutet, ein ein- oder mehrkerniges cycloaliphatisches Brückenglied mit 5 bis 12 Kohlenstoffatomen, ein ein- oder mehrkerniges aromatisches oder araliphatisches Brückenglied darstellt, wobei die aliphatischen, cycloaliphatischen, araliphatischen und aromatischen Brückenglieder durch Carbon- oder Sulfonsäuregruppen, deren Salze bzw. Amide und Ester, Alkyl($C_1$-$C_4$)-, Hydroxy-, Alkoxy($C_1$-$C_4$)gruppen, primäre, sekundäre oder tertiäre Aminogruppen sowie durch Fluor-, Chlor- oder Bromatome, die aliphatischen Brückenglieder bevorzugt durch Fluoratome, substituiert sein können und die genannten aliphatischen, cycloaliphatischen, araliphatischen oder aromatischen Brückenglieder ein oder mehrere Stickstoff- und/oder Sauerstoff- und/oder Schwefelatome enthalten können, und $W^1$aber auch eine direkte Bindung bedeuten kann,
$W^2$ als vierwertiges Brückenglied ein geradkettiges oder verzweigtes, gesättigtes oder ungesättigten

aliphatisches Brückenglied mit 2 bis 30 Kohlenstoffatomen, ein ein- oder mehrkerniges cycloaliphatisches Brückenglied mit 5 bis 12 Kohlenstoffatomen, ein ein- oder mehrkerniges aromatisches oder araliphatisches Brückenglied darstellt, wobei die aliphatischen, cycloaliphatischen, araliphatischen und aromatischen Brückenglieder durch Carbon- oder Sulfonsäuregruppen, deren Salze bzw. Amide und Ester, Alkyl($C_1$-$C_4$)-, Hydroxy-, Alkoxy($C_1$-$C_4$)gruppen, primäre, sekundäre oder tertiäre Aminogruppen sowie durch Fluor-, Chlor- oder Bromatome, die aliphatischen Brückenglieder bevorzugt durch Fluoratome, substituiert sein können, und die genannten aliphatischen Brückenglieder sowie die cycloaliphatischen, araliphatischen oder aromatischen Ringsysteme ein oder mehrere Stickstoff- und/oder Sauerstoff- und/oder Schwefelatome enthalten können,
und $W^3$ als dreiwertiges Brückenglied ein geradkettiges oder verzweigtes, gesättigtes oder ungesättigtes aliphatisches Brückenglied mit 2 bis 30 Kohlenstoffatomen, ein ein- oder mehrkerniges cycloaliphatisches Brückenglied mit 5 bis 12 Kohlenstoffatomen, ein ein- oder mehrkerniges aromatisches oder araliphatisches Brückenglied darstellt, wobei die aliphatischen, cycloaliphatischen, araliphatischen und aromatischen Brückenglieder durch Carbon- oder Sulfonsäuregruppen, deren Salze bzw. Amide und Ester, Alkyl($C_1$-$C_4$)-, Hydroxy-, Alkoxy($C_1$-$C_4$)gruppen, primäre, sekundäre oder tertiäre Aminogruppen sowie durch Fluor-,Chlor- oder Bromatome, die aliphatischen Brückenglieder bevorzugt durch Fluoratome, substituiert sein können, und die genannten aliphatischen, cycloaliphatischen, araliphatischen oder aromatischen Brückenglieder ein oder mehrere Stickstoff- und/oder Sauerstoff- und/oder Schwefelatome enthalten können.

**6.** Biskationische Säureamid- und -imidderivate nach mindestens einem der Ansprüche 1 bis 5, dadurch gekennzeichnet, daß in den in Anspruch 1 genannten Formeln (I) bis (III)
K und K' jeweils ein Stickstoffatom bedeuten,
$R_1$ bis $R_6$ entweder unabhängig voneinander jeweils ein Wasserstoffatom oder eine Alkyl($C_1$-$C_4$)gruppe darstellen oder $R_1$ und $R_2$ bzw. $R_4$ und $R_5$ sich unabhängig voneinander unter Einbeziehung von K bzw. K' zu einem gesättigten oder ungesättigten 5- oder 6-gliedrigen heterocyclischen Ringsystem mit einem oder zwei Stickstoffatom(en) als Heterobestandteil zusammenschließen können, wobei dann $R_3$ bzw. $R_6$ unabhängig voneinander jeweils ein Wasserstoffatom oder eine Alkyl($C_1$-$C_4$)gruppe darstellen und für den Fall, daß $R_1$ oder $R_2$ bzw. $R_4$ oder $R_5$ in diesem Zusammenhang eine Doppelbindung zu K oder K' ausbilden dann $R_3$ bzw. $R_6$ gegenstandslos sind,
und $R_7$ und $R_8$ jeweils ein Wasserstoffatom bedeuten und A und A' unabhängig voneinander jeweils ein $-(CH_2-)_p$-Brückenglied mit p = 1 bis 4, ein Phenylen-, Naphthylen- oder

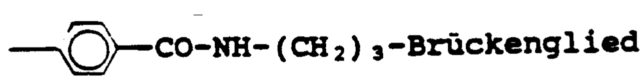

bedeuten, $W^1$ ein Phenylen-, Naphthylen-, Cyclohexylen- oder $-(CH_2)_q$-Brückenglied mit q = 1 bis 12 oder ein $-(CH_2-O-(CH_2-CH_2-O)_r-CH_2$-Brückenglied mit r = 1 bis 4, $W^2$ ein Ethylendiamintetramethylen-, ein Phenylen- oder Naphthylen-Brückenglied, wobei die zur Imidbildung benötigten Carboxylgruppen im Falle von Phenylen jeweils in ortno-Stellung, im Falle von Naphthylen in ortho- und/oder peri-Stellung zueinander stehen,
und $W^3$ ein Phenylen- oder Naphthylen-Brückenglied bedeuten, und die Reste $R_9$ bis $R_{12}$ Phenyl-, Naphthyl-, Fluorphenyl-, Chlorphenyl-, Methoxyphenyl-, Biphenyl-, Pyridyl-, Tolylreste oder Fluoratome bedeuten.

**7.** Biskationisches Säureamidderivat nach mindestens einem der Ansprüche 1 bis 6, gekennzeichnet durch die Formel (I)

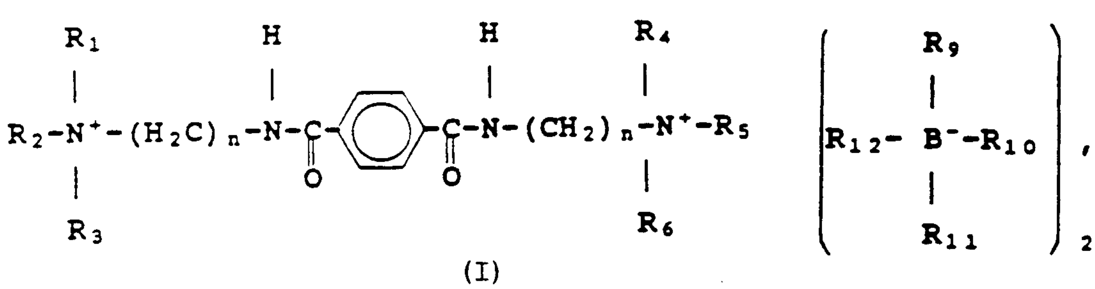

(I)

mit unabhängig voneinander $R_1,R_4$ = H, $CH_3$, $C_2H_5$, $R_2,R_3,R_5,R_6$ = $CH_3$, $C_2H_5$, n = 1 bis 5 und fur das Anion = $BF_4^-$ oder $B(Phenyl)_4^-$.

**8.** Biskationisches Säureamidderivat nach mindestens einem der Ansprüche 1 bis 6, gekennzeichnet durch die Formel (I)

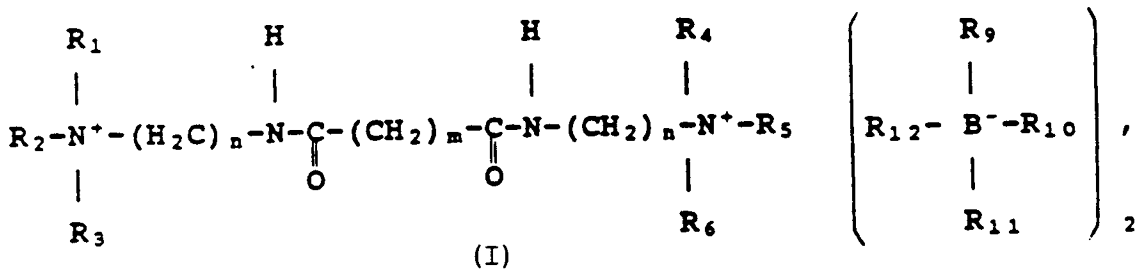

(I)

mit unabhängig voneinander $R_1,R_4$ = H, $CH_3$, $C_2H_5$, $R_2,R_3,R_5,R_6$ = $CH_3$, $C_2H_5$, m = 1 bis 8, n = 2 oder 3 und für das Anion = $BF_4^-$ oder $B(Phenyl)_4^-$.

**9.** Biskationisches Säureimidderivat nach mindestens einem der Ansprüche 1 bis 6, gekennzeichnet durch die Formel (II)

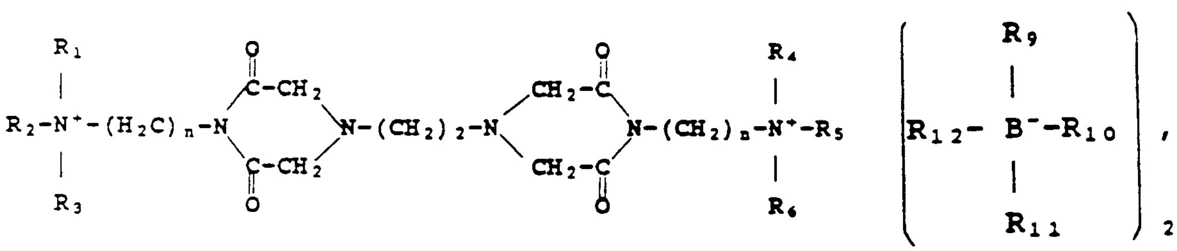

(II)

mit unabhängig voneinander $R_1,R_4$ = H, $CH_3$, $C_2H_5$, $R_2,R_3,R_5,R_6$ = $CH_3$, $C_2H_5$, n = 2 oder 3 und für das Anion = $BF_4^-$ oder $B(Phenyl)_4^-$.

**10.** Biskationisches Säureimidderivat nach mindestens einem der Ansprüche 1 bis 6, gekennzeichnet durch die Formel (II)

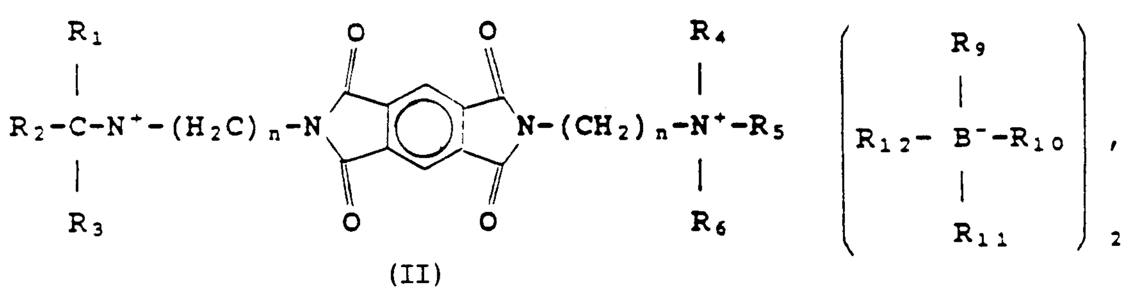

(II)

mit unabhängig voneinander $R_1,R_4$ = H, $CH_3$, $C_2H_5$, $R_2,R_3,R_5,R_6$ = $CH_3$, $C_2H_5$, n = 2 oder 3 und für das Anion = $BF_4^-$ oder $B(Phenyl)_4^-$.

**11.** Biskationisches Säureimidderivat nach mindestens einem der Ansprüche 1 bis 6, gekennzeichnet durch die Formel (II)

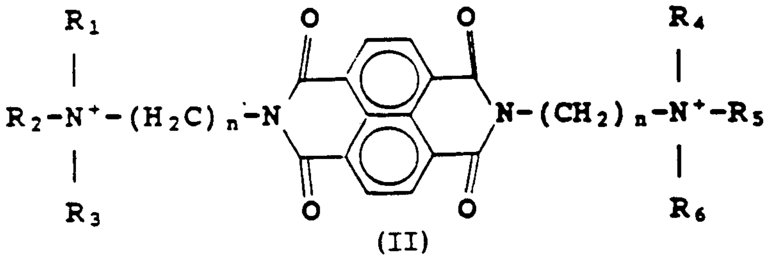 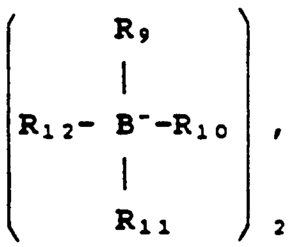 ,

(II)

mit unabhängig voneinander $R_1$,$R_4$ = H, $CH_3$, $C_2H_5$, $R_2$,$R_3$,$R_5$,$R_6$ = $CH_3$, $C_2H_5$, n = 2 oder 3 und für das Anion = $BF_4^-$ oder $B(Phenyl)_4^-$.

**12.** Biskationisches Säureamidderivat nach mindestens einem der Ansprüche 1 bis 6, gekennzeichnet durch die Formel (I)

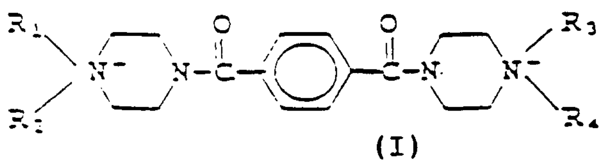 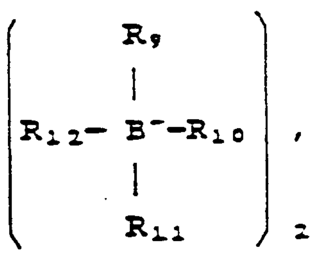 ,

(I)

mit unabhängig voneinander $R_1$, $R_3$ = H, $CH_3$, $C_2H_5$, $R_2$, $R_4$ = $CH_3$, $C_2H_5$ und für das Anion = $BF_4^-$ oder $B(Phenyl)_4^-$.

**13.** Verfahren zur Herstellung von Säureamid- und -imidderivaten bzw. Gemischen aus Säureamid- und -imidderivaten oder Mischkristallen mit gemischten Anionen und/oder Kationen der in Anspruch 1 genannten allgemeinen Formeln (I) bis (III), dadurch gekennzeichnet, daß man Säureamid- und -imidderivate der allgemeinen Formeln (IV) bis (VI)

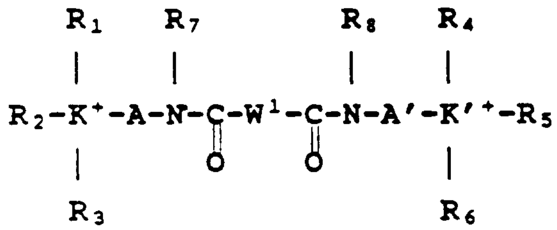

2 $X^-$,

(IV)

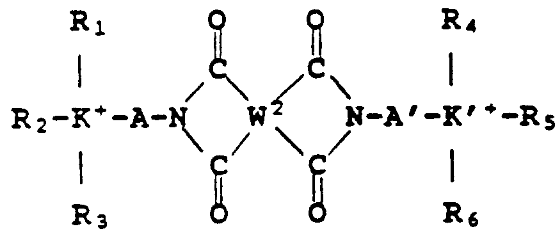

2 $X^-$,

(V)

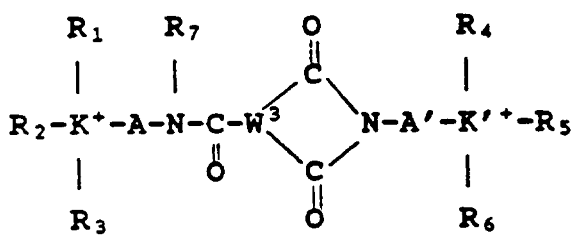

2 $X^-$,

(VI)

wobei $R_1$ bis $R_8$, A und A', K und K' sowie $W^1$, $W^2$ und $W^3$ die in den Ansprüchen 1 bis 11 genannten Bedeutungen haben und $X^-$ das stöchiometrische Äquivalent eines oder mehrerer Halogenanion(en), Methylsulfat-, Ethylsulfat-, Hydrogensulfat- oder Sulfatanion(en) bedeutet, mit einem oder mehreren der in Anspruch 1, in den Ansprüchen 6 bis 11 und in Anspruch 14 genannten Boratsalze in Wasser oder Gemischen aus Wasser und Säure oder Gemischen aus Wasser und organischem Lösungsmittel bei Temperaturen von etwa 10 °C bis etwa 90 °C umsetzt.

**14.** Verfahren nach Anspruch 13 dadurch gekennzeichnet, daß man die in Anspruch 12 genannten Verbindungen der allgemeinen Formeln (IV) bis (VI) in einem Gemisch aus Wasser und Essigsäure, Isopropanol, Isobutanol oder Methylisobutylketon umsetzt.

**15.** Verfahren nach mindestens einem der Ansprüche 13 und 14 dadurch gekennzeichnet, daß man die in Anspruch 12 genannten Verbindungen der allgemeinen Formeln (IV) bis (VI) mit Natriumtetraphenylborat, Natriumtetra-o-fluorphenylborat, Natriumtetra-m-fluorphenylborat, Natriumtetra-p-fluorphenylborat, Natriumtetra-o-chlorphenylborat, Natriumtetra-m-chlorphenylborat, Natriumtetra-p-chlorphenylborat, Natriumtetra-o-tolylborat, Natriumtetra-m-tolylborat, Natriumtetra-p-tolylborat, Natriumtetra-1-naphthylborat, Natriumtetra-2-naphthylborat, Natriumtetra-o-methoxyphenylborat, Natriumtetra-m-methoxyhenylborat,

Natriumtetra-p-methoxyphenylborat, Natriumtetra-o-biphenylborat, Natriumtetra-m-biphenylborat, Natriumtetra-p-biphenylborat, Natriumtetrabenzylborat, Natriumtetra-o-pyridylborat, Natriumtetra-m-pyridylborat, Natriumtetra-p-pyridylborat oder Natriumtetrafluoroborat umsetzt.

## Claims

**1.** Biscationic acid amide and imide derivatives of the general formulae (I) to (III)

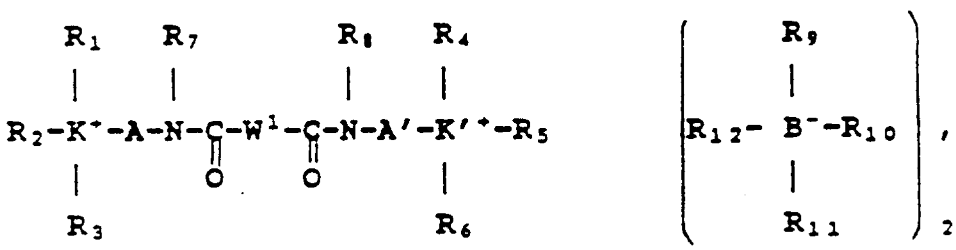

(I)

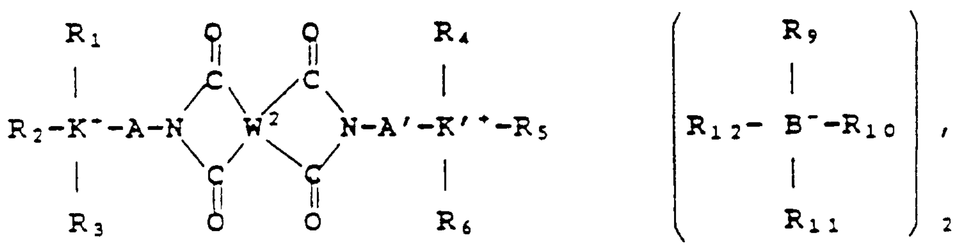

(II)

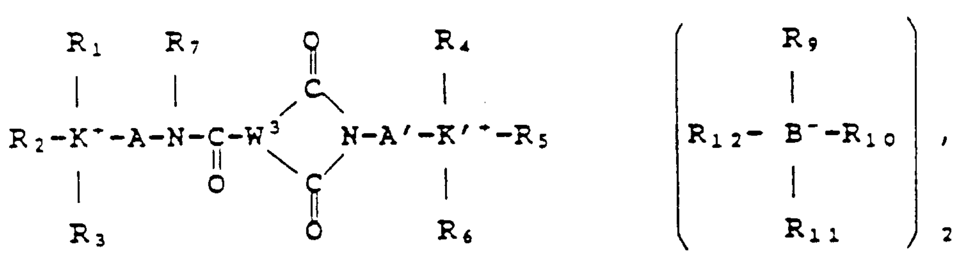

(III)

in which $R_1$ to $R_8$ independently of one another are in each case a hydrogen atom or in each case a radical based on a hydrocarbon, which can be interrupted by hetero atoms, in which the radicals $R_1$ and $R_2$ or $R_4$ and $R_5$ independently of one another, incorporating K or K', can be closed together to form a ring system, and in the case where $R_1$ or $R_2$, or $R_4$ or $R_5$ in this connection form a double bond to K or K', $R_3$ or $R_6$ has no subject, and/or one of the radicals $R_1$, $R_2$ or $R_3$ can be closed together with $R_7$ or one of the radicals $R_4$, $R_5$ or $R_6$ can be closed together with $R_8$ to form an aliphatic bridge of 2 to 5 carbon atoms,

and in which A and A' and $W^1$ as a divalent, $W^2$ as a tetravalent and $W^3$ as a trivalent bridge member independently of one another are in each case a bridge member based on a hydrocarbon, which can be interrupted by hetero atoms, in which $W^1$ can also be a direct bond,

and K and K' independently of one another are in each case a nitrogen, phosphorus, arsenic or antimony atom, and the radicals $R_9$ to $R_{12}$ independently of one another are aliphatic, cycloaliphatic, iso- or heterocyclic, aromatic or araliphatic radicals, in which the aliphatic, cycloaliphatic, aromatic and araliphatic radicals mentioned can be substituted by alkyl($C_1$-$C_4$), alkoxy($C_1$-$C_4$) or aryl radicals or halogen atoms, or are fluorine atoms, and mixtures of these compounds and mixed crystals with mixed anions and/or cations.

2. Biscationic acid amide and imide derivatives according to claim 1, characterized in that, in the formulae (I) to (III) mentioned in claim 1, K and K' are in each case a nitrogen atom.

3. Biscationic acid amide and imide derivatives according to at least one of claims 1 to 2, characterized in that, in the formulae (I) to (III) mentioned in claim 1, $R_1$ to $R_8$ independently of one another are in each case a hydrogen atom, a straight-chain or branched, saturated or unsaturated alkyl group having 1 to 30 carbon atoms, a polyoxyalkylene group of the general formula -[alkylene($C_1$-$C_5$)-O]$_n$-R, in which R is a hydrogen atom, an alkyl($C_1$-$C_4$) group or an acyl group and n is a number from 1 to 10, a mono- or polynuclear cycloaliphatic radical having 5 to 12 carbon atoms or a mono- or polynuclear aromatic or araliphatic radical, in which such aliphatic, cycloaliphatic, araliphatic and aromatic radicals can be substituted by carboxylic or sulfonic acid groups, salts or amides or esters thereof, alkyl($C_1$-$C_4$), hydroxyl or alkoxy($C_1$-$C_4$) groups, primary, secondary or tertiary amino groups and by fluorine, chlorine or bromine atoms, the aliphatic radicals preferably by fluorine atoms, and in which the aliphatic radicals mentioned and the cycloaliphatic, araliphatic or aromatic ring systems can contain one or more hetero atoms and $R_1$ and $R_2$, or $R_4$ and $R_5$ independently of one another, incorporating K or K', can be closed together to form a saturated or unsaturated, aromatic or non-aromatic 5- to 7-membered ring system, which can contain further hetero atoms and can be substituted and/or modified by fusion with or bridging to further ring systems, and in which, in the case where $R_1$ or $R_2$, or $R_4$ or $R_5$ in this connection form a double bond to K or K', $R_3$ or $R_6$ has no subject.

4. Biscationic acid amide and imide derivatives according to at least one of claims 1 to 3, characterized in that, in the formulae (I) to (III) mentioned in claim 1,
A and A' independently of one another are in each case a straight-chain or branched, saturated or unsaturated aliphatic bridge member having 1 to 30 carbon atoms, a mono- or polynuclear cycloaliphatic bridge member or a mono- or polynuclear aromatic or araliphatic bridge member, in which such aliphatic, cycloaliphatic, araliphatic and aromatic bridge members can be substituted by carboxylic or sulfonic acid groups, salts or amides or esters thereof, alkyl($C_1$-$C_4$), hydroxyl or alkoxy-($C_1$-$C_4$) groups, primary, secondary or tertiary amino groups and by fluorine, chlorine or bromine atoms, the aliphatic bridge members preferably by fluorine atoms, and the aliphatic, cycloaliphatic, araliphatic or aromatic bridge members mentioned can contain one or more nitrogen and/or oxygen and/or sulfur atoms.

5. Biscationic acid amide and imide derivatives according to at least one of claims 1 to 4, characterized in that, in the formulae (I) to (III) mentioned in claim 1,
$W^1$ as a divalent bridge member is a straight-chain or branched, saturated or unsaturated aliphatic intermediate member having 1 to 30 carbon atoms, a polyoxyalkylene member of the general formula -[$CH_2$-O-(alkylene($C_1$-$C_5$)-O]$_m$-$CH_2$-, in which m is a number from 0 to 10, a mono- or polynuclear cycloaliphatic bridge member having 5 to 12 carbon atoms or a mono- or polynuclear aromatic or araliphatic bridge member, in which the aliphatic, cycloaliphatic, araliphatic and aromatic bridge members can be substituted by carboxylic or sulfonic acid groups, salts or amides or esters thereof, alkyl($C_1$-$C_4$), hydroxyl or alkoxy($C_1$-$C_4$) groups, primary, secondary or tertiary amino groups and by fluorine, chlorine or bromine atoms, the aliphatic bridge members preferably by fluorine atoms, and the aliphatic, cycloaliphatic, araliphatic or aromatic bridge members mentioned can contain one or more nitrogen and/or oxygen and/or sulfur atoms, but $W^1$ can also be a direct bond,
$W^2$ as a tetravalent bridge member is a straight-chain or branched, saturated or unsaturated aliphatic bridge member having 2 to 30 carbon atoms, a mono- or polynuclear cycloaliphatic bridge member having 5 to 12 carbon atoms or a mono- or polynuclear aromatic or araliphatic bridge member, in which the aliphatic, cycloaliphatic, araliphatic and aromatic bridge members can be substituted by carboxylic or sulfonic acid groups, salts or amides or esters thereof, alkyl($C_1$-$C_4$), hydroxyl or alkoxy($C_1$-$C_4$) groups, primary, secondary or tertiary amino groups and by fluorine, chlorine or bromine atoms, the aliphatic bridge members preferably by fluorine atoms, and the aliphatic bridge members mentioned

and the cycloaliphatic, araliphatic or aromatic ring systems can contain one or more nitrogen and/or oxygen and/or sulfur atoms,
and $W^3$ as a trivalent bridge member is a straight-chain or branched, saturated or unsaturated aliphatic bridge member having 2 to 30 carbon atoms, a mono- or polynuclear cycloaliphatic bridge member having 5 to 12 carbon atoms or a mono- or polynuclear aromatic or araliphatic bridge member, in which the aliphatic, cycloaliphatic, araliphatic and aromatic bridge members can be substituted by carboxylic or sulfonic acid groups, salts and amides and esters thereof, alkyl($C_1$-$C_4$), hydroxyl or alkoxy($C_1$-$C_4$) groups, primary, secondary or tertiary amino groups and by fluorine, chlorine or bromine atoms, the aliphatic bridge members preferably by fluorine atoms, and the aliphatic, cycloaliphatic, araliphatic or aromatic bridge members mentioned can contain one or more nitrogen and/or oxygen and/or sulfur atoms.

**6.** Biscationic acid amide and imide derivatives according to at least one of claims 1 to 5, characterized in that, in the formulae (I) to (III) mentioned in claim 1,
K and K' are in each case a nitrogen atom,
$R_1$ to $R_6$ either independently of one another are in each case a hydrogen atom or an alkyl($C_1$-$C_4$) group, or $R_1$ and $R_2$, or $R_4$ and $R_5$ independently of one another, incorporating K or K', can be closed together to form a saturated or unsaturated 5- or 6-membered heterocyclic ring system having one or two nitrogen atom(s) as the hetero constituent, in which $R_3$ or $R_6$ independently of one another are then in each case a hydrogen atom or an alkyl($C_1$-$C_4$) group, and in the case where $R_1$ or $R_2$, or $R_4$ or $R_5$ in this connection forms a double bond to K or K', $R_3$ and $R_6$ have no subject,
and $R_7$ and $R_8$ are in each case a hydrogen atom,
and A and A' independently of one another are in each case a $-(CH_2-)_p-$ bridge member, where p is 1 to 4, or a phenylene, naphthylene or

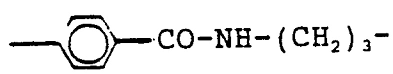

bridge member,
$W^1$ is a phenylene, naphthylene, cyclohexylene or $-(CH_2)_q-$bridge member, where q is 1 to 12, or a $-(CH_2-O-(CH_2-CH_2-O)_r-CH_2-$ bridge member, where r is 1 to 4, $W^2$ is an ethylenediaminetetramethylene, a phenylene or a naphthylene bridge member, in which the carboxyl groups required for imide formation are in each case in the ortho-position relative to one another in the case of phenylene and in the ortho- and/or peri-position relative to one another in the case of naphthylene,
and $W^3$ is a phenylene or naphthylene bridge member,
and the radicals $R_9$ to $R_{12}$ are phenyl, naphthyl, fluorophenyl, chlorophenyl, methoxyphenyl, biphenyl, pyridyl or tolyl radicals or fluorine atoms.

**7.** Biscationic acid amide derivative according to at least one of claims 1 to 6, characterized by the formula (I)

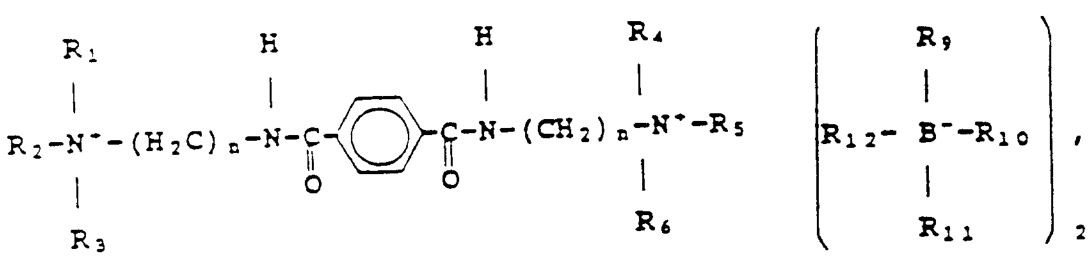

(I)

in which, independently of one another, $R_1$ and $R_4$ are H, $CH_3$ or $C_2H_5$, $R_2$, $R_3$, $R_5$ and $R_6$ are $CH_3$ or $C_2H_5$, n is 1 to 5 and for the anion = $BF_4^-$ or $B(phenyl)_4^-$.

**8.** Biscationic acid amide derivative according to at least one of claims 1 to 6, characterized by the formula (I)

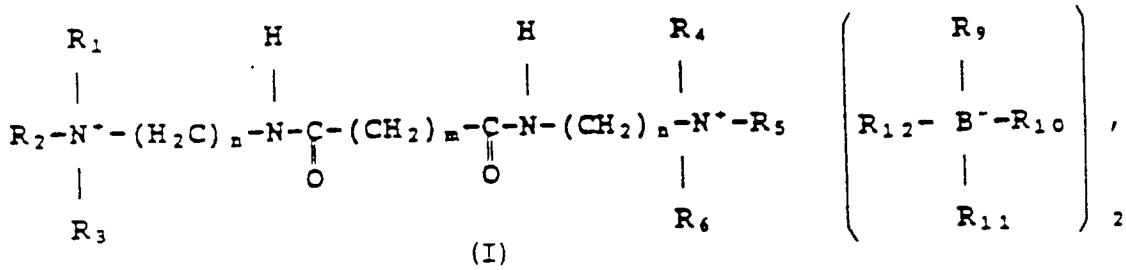

(I)

in which, independently of one another, $R_1$ and $R_4$ are H, $CH_3$ or $C_2H_5$, $R_2$, $R_3$, $R_5$ and $R_6$ are $CH_3$ or $C_2H_5$, m is 1 to 8, n is 2 or 3 and for the anion = $BF_4^-$ or $B(phenyl)_4^-$.

**9.** Biscationic acid imide derivative according to at least one of claims 1 to 6, characterized by the formula (II)

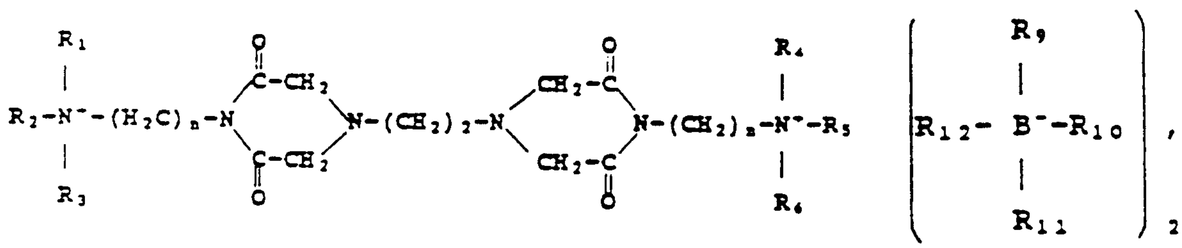

(II)

in which, independently of one another, $R_1$ and $R_4$ are H, $CH_3$ or $C_2H_5$, $R_2$, $R_3$, $R_5$ and $R_6$ are $CH_3$ or $C_2H_5$, n is 2 or 3 and for the anion = $BF_4^-$ or $B(phenyl)_4^-$.

**10.** Biscationic acid imide derivative according to at least one of claims 1 to 6, characterized by the formula (II)

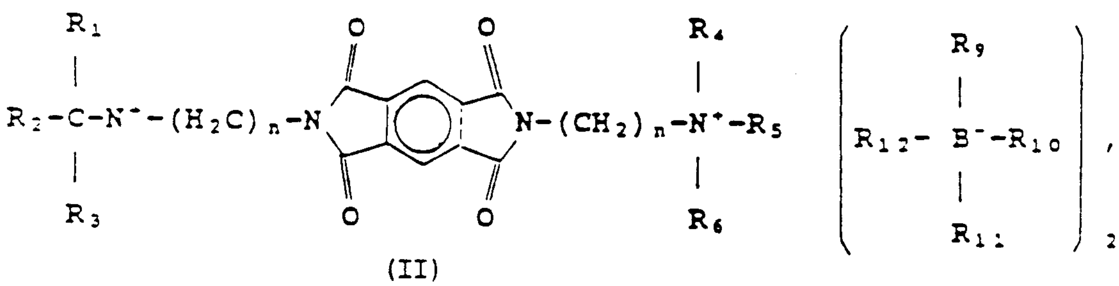

(II)

in which, independently of one another, $R_1$ and $R_4$ are H, $CH_3$ or $C_2H_5$, $R_2$, $R_3$, $R_5$ and $R_6$ are $CH_3$ or $C_2H_5$, n is 2 or 3 and for the anion = $BF_4^-$ or $B(phenyl)_4^-$.

**11.** Biscationic acid imide derivative according to at least one of claims 1 to 6, characterized by the formula (II)

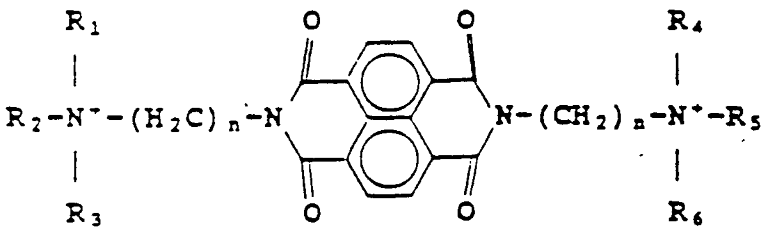

(II)

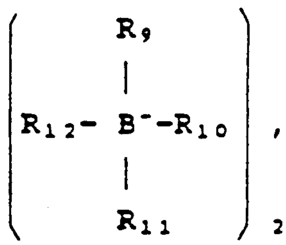

in which, independently of one another, $R_1$ and $R_4$ are H, $CH_3$ or $C_2H_5$, $R_2$, $R_3$, $R_5$ and $R_6$ are $CH_3$ or $C_2H_5$, n is 2 or 3 and for the anion = $BF_4^-$ or $B(phenyl)_4^-$.

**12.** Biscationic acid amide derivative according to at least one of claims 1 to 6, characterized by the formula (I)

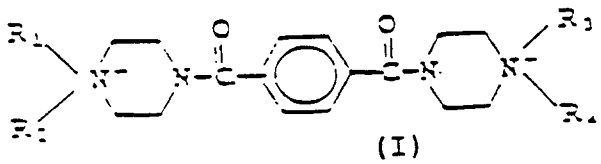

(I)

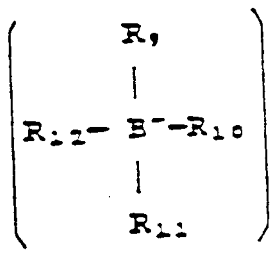

in which, independently of one another, $R_1$ and $R_3$ are H, $CH_3$ or $C_2H_5$, $R_2$ and $R_4$ are $CH_3$ or $C_2H_5$ and for the anion = $BF_4^-$ or $B(phenyl)_4^-$.

**13.** Process for the preparation of acid amide and imide derivatives or mixtures of acid amide and imide derivatives or mixed crystals with mixed anions and/or cations, of the general formulae (I) to (III) mentioned in claim 1, characterized in that acid amide or imide derivatives of the general formulae (IV) to (VI)

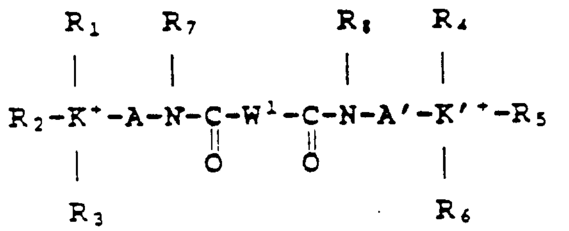

2 $X^-$,

(IV)

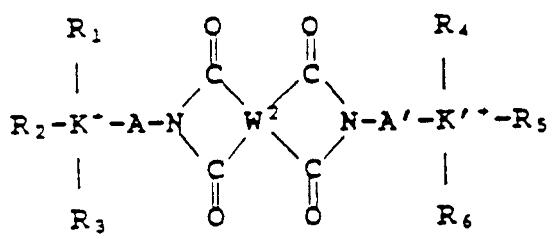

2 $X^-$,

(V)

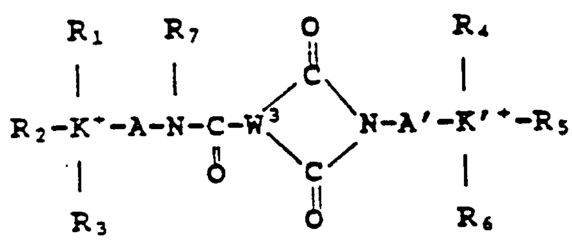

2 $X^-$,

(VI)

in which $R_1$ to $R_8$, A and A', K and K' and $W^1$, $W^2$ and $W^3$ have the meanings given in claims 1 to 11 and $X^-$ is the stoichiometric equivalent of one or more halogen anion(s) or methyl-sulfate, ethyl-sulfate, hydrogen sulfate or sulfate anion(s), are reacted with one or more of the borate salts mentioned in claim 1, in claims 6 to 11 and in claim 15 in water or mixtures of water and acid or mixtures of water and organic solvent at temperatures of about 10 °C to about 90 °C.

**14.** Process according to claim 13, characterized in that the compounds of the general formulae (IV) to (VI) mentioned in claim 13 are reacted in a mixture of water and acetic acid, isopropanol, isobutanol or methyl isobutyl ketone.

**15.** Process according to at least one of claims 13 and 14, characterized in that the compounds of the general formulae (IV) to (VI) mentioned in claim 13 are reacted with sodium tetraphenylborate, sodium tetra-o-fluorophenylborate, sodium tetra-m-fluorophenylborate, sodium tetra-p-fluorophenylborate, sodium tetra-o-chlorophenylborate, sodium tetra-m-chlorophenylborate, sodium tetra-p-chlorophenylborate, sodium tetra-o-tolylborate, sodium tetra-m-tolylborate, sodium tetra-p-tolylborate, sodium tetra-1-naphthylborate, sodium tetra-2-naphthylborate, sodium tetra-o-methoxyphenylborate, sodium tetra-m-methoxyphenylborate, sodium tetra-p-methoxyphenylborate, sodium tetra-o-biphenylborate, sodium tetra-m-biphenylborate, sodium tetra-p-biphenylborate, sodium tetrabenzylborate, sodium tetra-o-pyridylborate, sodium tetra-m-pyridylborate, sodium tetra-p-pyridylborate or sodium tetrafluoroborate.

## Revendications

**1.** Amides et imides bis-cationiques de formules générales I à III ci-dessous, mélanges de ces composés et cristaux mixtes à anions et/ou cations mixtes :

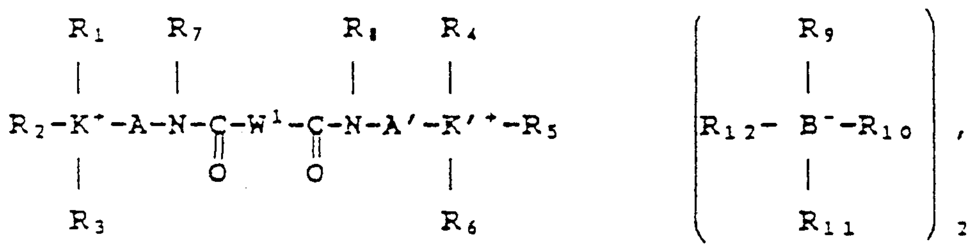

(I)

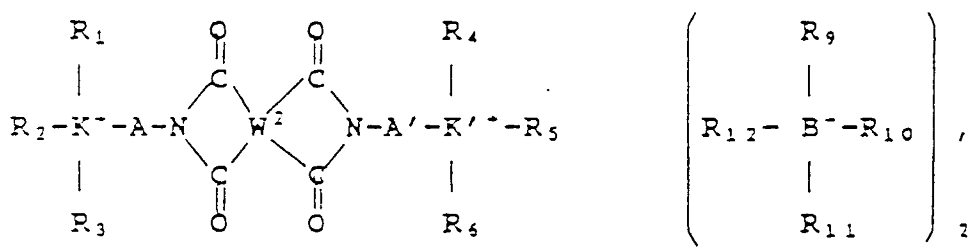

(II)

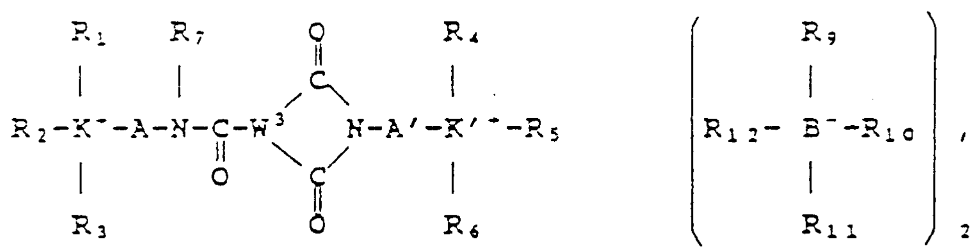

(III)

formules dans lesquelles

$R_1$ à $R_8$ représentent chacun, indépendamment les uns des autres, un atome d'hydrogène ou un radical d'hydrocarbure pouvant être interrompu par des hétéroatomes,

les radicaux $R_1$ et $R_2$ et/ou $R_4$ et $R_5$ pouvant être cyclisés en des systèmes cycliques avec respectivement K et K' et si $R_1$ et $R_2$ et/ou $R_4$ ou $R_5$ forment alors une double liaison à K ou K', il n'y a plus de $R_3$ ou $R_6$, et/ou l'un des radicaux $R_1$ et $R_2$ ou $R_3$ avec $R_7$, ou l'un des radicaux $R_4$ et $R_5$ ou $R_6$ avec $R_8$, pouvant être liés en ponts aliphatiques de 2 à 5 atomes de carbone,

A et A' et $W^1$ comme ponts divalents, $W^2$ comme pont tétravalent et $W^3$ comme pont trivalent représentent chacun indépendamment un pont hydrocarboné pouvant être interrompu par des hétéroatomes, $W^1$ pouvant être aussi une liaison directe,

K et K', indépendamment l'un de l'autre, représentent chacun un atome d'azote, de phosphore, d'arsenic ou d'antimoine,

et les radicaux $R_9$ à $R_{12}$, indépendamment les uns des autres, représentent des radicaux aliphatiques, cycloaliphatiques, aromatiques ou araliphatiques isocycliques ou hétérocycliques, les radicaux aliphatiques, cycloaliphatiques, aromatiques et araliphatiques pouvant être substitués par des alkyles ou des alcoxy en $C_1$-$C_4$,par des aryles ou des atomes d'halogènes, ou bien représentent des atomes de fluor.

**2.** Amides et imides bis-cationiques selon la revendication 1, caractérisés en ce que dans les formules I à III K et K' sont chacun un atome d'azote.

**3.** Amides et imides bis-cationiques selon la revendication 1 et/ou 2, caractérisés en ce que dans les formules I à III $R_1$ à $R_8$ sont indépendamment des atomes d'hydrogène, des alkyles linéaires ou ramifiés, saturés ou insaturés, pouvant avoir de 1 à 30 atomes de carbone, des polyoxalkylènes [alkylène($C_1$-$C_5$)-O]$_n$- R R étant un atome d'hydrogène, un alkyle en $C_1$-$C_4$ ou un acyle et n un nombre de 1 à 10, des radicaux cycloaliphatiques monocycliques ou polycycliques ayant de 5 à 12 atomes de carbone ou des radicaux aromatiques ou araliphatiques monocycliques ou polycycliques, radicaux aliphatiques, cycloaliphatiques, araliphatiques et aromatiques qui peuvent être substitués par des groupes carboxyliques ou sulfoniques, leurs sels ou amides et esters, des alkyles et alcoxy en $C_1$-$C_4$, des hydroxyles, des groupes amino primaires, secondaires ou tertiaires, ainsi que par des atomes de fluor, de chlore ou de brome, les radicaux aliphatiques ayant de préférence des atomes de fluor, et les radicaux aliphatiques ainsi que les systèmes cycliques cycloaliphatiques, araliphatiques ou aromatiques peuvent comporter un ou plusieurs hétéro-atomes, $R_1$ et $R_2$ et/ou $R_4$ et $R_5$, indépendamment et avec K ou K', peuvent être liés en des systèmes cycliques saturés ou insaturés, aromatiques ou non aromatiques, pentagonaux à heptagonaux, qui peuvent comprendre encore d'autres hétéroatomes et substituants et/ou qui peuvent être modifiés par condensation de ou pontage à d'autres systèmes cycliques, et si $R_1$ ou $R_2$ et/ou $R_4$ ou $R_5$ forment à cet égard une double liaison respectivement à K et K', alors il n'y a plus de $R_3$ ou de $R_6$.

**4.** Amides et imides bis-cationiques selon une ou plusieurs des revendications 1 à 3, caractérisés en ce que dans les formules I à III A et A' sont chacun indépendamment un pont aliphatique avec de 1 à 30 atomes de carbone, linéaire ou ramifié, saturé ou insaturé, un pont cycloaliphatique monocyclique ou polycyclique ou encore un pont aromatique ou araliphatique monocyclique ou polycyclique, ponts aliphatiques, cycloaliphatiques, araliphatiques et aromatiques qui peuvent être substitués par des groupes carboxyliques ou sulfoniques, leurs sels ou amides et esters, des alkyles et alcoxy en $C_1$-$C_4$, des hydroxyles, des groupes amino primaires, secondaires ou tertiaires, ainsi que par des atomes de fluor, de chlore ou de brome, les radicaux aliphatiques ayant de préférence des atomes de fluor, et les ponts aliphatiques, cycloaliphatiques,

araliphatiques ou aromatiques peuvent également comprendre un ou plusieurs atomes d'azote et/ou de soufre et/ou d'oxygène.

**5.** Amides et imides bis-cationiques selon une ou plusieurs des revendications 1 à 4, caractérisés en ce que dans les formules I à III $W^1$ est en tant que pont divalent un pont aliphatique ayant de 1 à 30 atomes de carbone, linéaire ou ramifié, saturé ou insaturé, un pont polyoxalkylène -[$CH_2$-O-(alkylène-($C_1$-$C_5$)-O]$_m$-$CH_2$, m étant un nombre de 0 à 10, un pont cycloaliphatique monocyclique ou polycyclique avec de 5 à 12 atomes de carbone, ou bien un pont aromatique ou araliphatique monocyclique ou polycyclique, ponts aliphatiques, cycloaliphatiques, araliphatiques et aromatiques qui peuvent être substitués par des groupes carboxyliques et sulfoniques, leurs sels ou amides ou esters, par des alkyles ou alcoxy en $C_1$-$C_4$, des hydroxyles, des groupes amino primaires, secondaires ou tertiaires, ainsi que par des atomes de fluor, de chlore et de brome, les ponts aliphatiques ayant de préférence des atomes de fluor comme substituants, et les ponts aliphatiques, cycloaliphatiques, araliphatiques ou aromatiques peuvent comporter un ou plusieurs atomes d'azote et/ou d'oxygène et/ou de soufre, mais $W^1$ pouvant représenter aussi une liaison directe,

$W^{2,}$ en tant que pont tétravalent, est un pont aliphatique linéaire ou ramifié, saturé ou insaturé, ayant de 2 à 30 atomes de carbone, un pont cycloaliphatique monocyclique ou polycyclique avec de 5 à 12 atomes de carbone, ou bien un pont aromatique monocyclique ou polycyclique, ponts aliphatiques, cycloaliphatiques, araliphatiques et aromatiques qui peuvent être substitués par des groupes carboxyliques et sulfoniques, leurs sels ou amides et esters, par des alkyles ou alcoxy en $C_1$-$C_4$, des

hydroxyles, des groupes amino primaires, secondaires ou tertiaires, ainsi que par des atomes de fluor, de chlore et de brome, les ponts aliphatiques ayant de préférence des atomes de fluor comme substituants, et les ponts aliphatiques ainsi que les systèmes cycliques cycloaliphatiques, araliphatiques ou aromatiques peuvent comprendre un ou plusieurs atomes d'azote et/ou d'oxygène et/ou de soufre,

et $W^{3,}$ en tant que pont trivalent, est un pont aliphatique linéaire ou ramifié, saturé ou insaturé, ayant de 2 à 30 atomes de carbone, un pont cycloaliphatique monocyclique ou polycyclique avec de 5 à 12 atomes de carbone, ou encore un pont aromatique monocyclique ou polycyclique, ponts aliphatiques, cycloaliphatiques, araliphatiques et aromatiques qui peuvent être également substitués par des groupes carboxyliques et sulfoniques, leurs sels ou amides et esters, par des alkyles et alcoxy en $C_1$-$C_4$, des hydroxyles, des groupes amino primaires, secondaires ou tertiaires, ainsi que par des atomes de fluor, de chlore et de brome, les ponts aliphatiques ayant de préférence des atomes de fluor comme substituants, et les ponts aliphatiques, cycloaliphatiques, araliphatiques ou aromatiques peuvent également comporter un ou plusieurs atomes d'azote et/ou d'oxygène et/ou de soufre.

**6.** Amides et imides bis-cationiques selon une ou plusieurs des revendications 1 à 5, caractérisés en ce que dans les formules I à III K et K' représentent chacun un atome d'azote, $R_1$ à $R_6$ sont chacun indépendamment les uns des autres un atome d'hydrogène ou un alkyle en $C_1$-$C_4$ oubien $R_1$ ou $R_2$ et/ou $R_4$ ou $R_5$ peuvent être liés ensemble et avec respectivement K et K' en des systèmes cycliques saturés ou insaturés, pentagonaux ou hexagonaux, ayant un ou deux atomes d'azote comme hétéroatomes, auquel cas $R_3$ et $R_6$ sont des atomes d'hydrogène ou des alkyles en $C_1$-$C_4$, et si $R_1$ ou $R_2$ et/ou $R_4$ ou $R_5$ forment alors une double liaison respectivement à K et K', il n'y a plus de $R_3$ ni de $R_6$, $R_7$ et $R_8$ sont chacun un atome d'hydrogène, A et A' sont chacun indépendamment un pont $-(CH_2-)_p$, p étant un nombre de 1 à 4, ou bien un pont phénylène, naphtylène ou

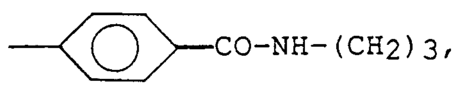

$W^1$ est un pont phénylène, naphtylène, cyclohexylène ou $-(CH_2-)_q$, q étant un nombre de 1 à 12, ou bien un pont $-CH_2-O-(CH_2-CH_2-O)_r-CH_2-$, r étant un nombre de 1 à 4,
$W^2$ est un pont éthylène-diamino-tétraméthylène, phénylène ou naphtylène, les groupes carboxyliques nécessaires à la formation de l'imide étant en position ortho l'un par rapport à l'autre dans le cas d'un phénylène et en position ortho et/ou péri dans le cas d'un naphtylène,
$W^3$ est un pont phénylène ou naphtylène,
et les radicaux $R_9$ à $R_{12}$ sont des radicaux phényle, naphtyle, fluorophényle, chlorophényle, méthoxyphényle, biphényle, pyridyle ou tolyle ou encore des atomes de fluor.

**7.** Amides bis-cationiques selon une ou plusieurs des revendications 1 à 6, caractérisés par la formule I

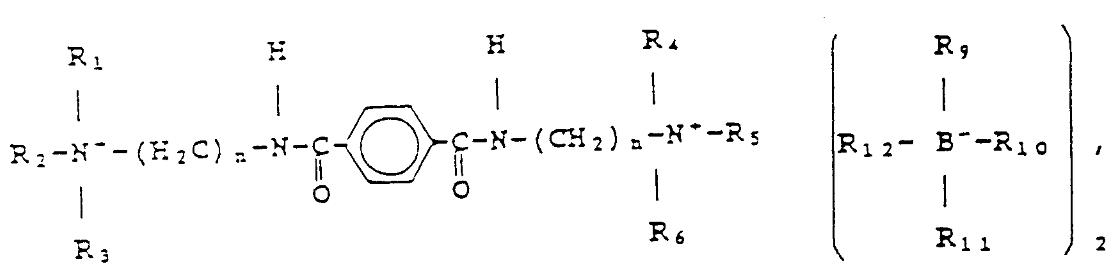

(I)

$R_1$ et $R_4$ représentant chacun indépendamment H, $CH_3$ ou $C_2H_5$, $R_2$, $R_3$, $R_5$ et $R_6$ représentant $CH_3$ ou $C_2H_5$, n un nombre de 1 à 5 et l'anion étant l'anion $BF_4^-$ ou $B(phényle)_4^-$.

**8.** Amides bis-cationiques selon une ou plusieurs des revendications 1 à 6, caractérisés par la formule I

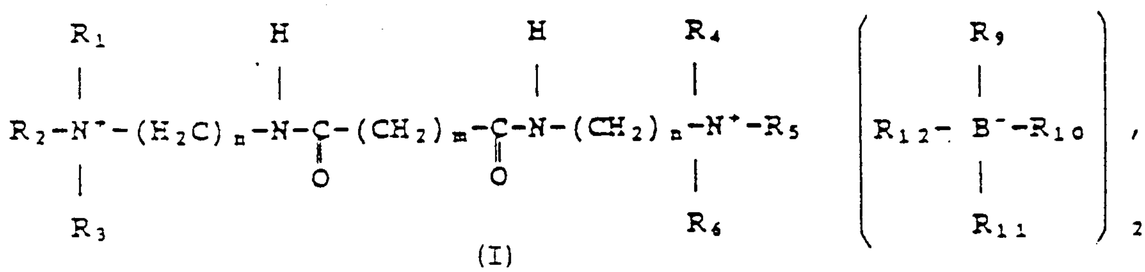

(I)

$R_1$ et $R_4$ représentant chacun indépendamment H, $CH_3$ ou $C_2H_5$, $R_2$, $R_3$, $R_5$ et $R_6$ représentant $CH_3$ ou $C_2H_5$, m un nombre de 1 à 8, n le nombre 2 ou 3 et l'anion étant l'anion $BF_4^-$ ou B(phényle)$_4^-$.

**9.** Imides bis-cationiques selon une ou plusieurs des revendications 1 à 6, caractérisés par la formule II

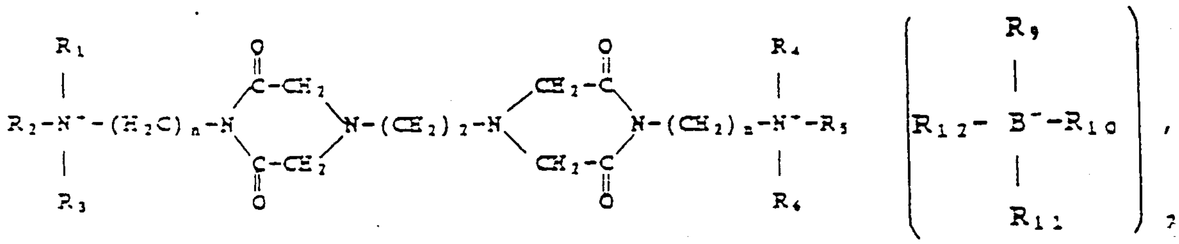

(II)

$R_1$ et $R_4$ représentant chacun indépendamment H, $CH_3$ ou $C_2H_5$, $R_2$, $R_3$, $R_5$ et $R_6$ représentant $CH_3$ ou $C_2H_5$, n le nombre 2 ou 3 et l'anion étant l'anion $BF_4^-$ ou B(phényle)$_4^-$.

**10.** Imides bis-cationiques selon une ou plusieurs des revendications 1 à 6, caractérisés par la formule II

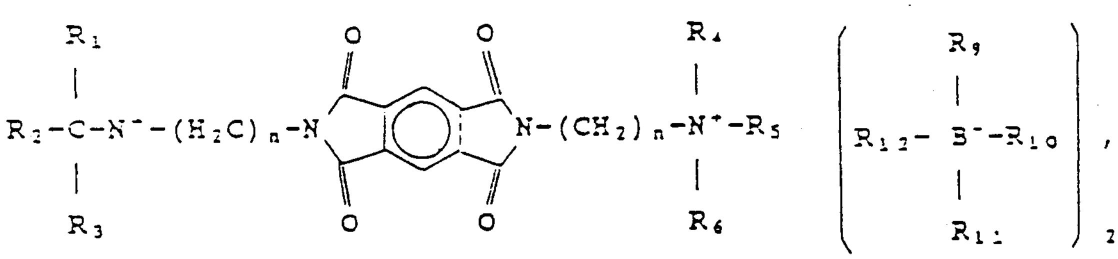

(II)

$R_1$ et $R_4$ représentant chacun indépendamment H, $CH_3$ ou $C_2H_5$, $R_2$, $R_3$, $R_5$ et $R_6$ représentant $CH_3$ ou $C_2H_5$, n le nombre 2 ou 3 et l'anion étant l'anion $BF_4^-$ ou B(phényle)$_4^-$.

**11.** Imides bis-cationiques selon une ou plusieurs des revendications 1 à 6, caractérisés par la formule II

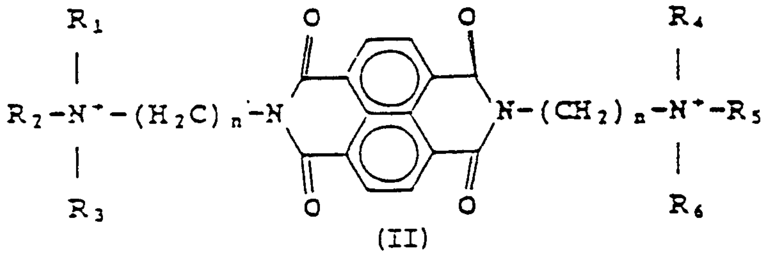

(II)

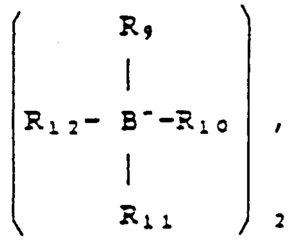

$R_1$ et $R_4$ représentant chacun indépendamment H, $CH_3$ ou $C_2H_5$, $R_2$, $R_3$, $R_5$ et $R_6$ représentant $CH_3$ ou $C_2H_5$, n le nombre 2 ou 3 et l'anion étant l'anion $BF_4^-$ ou $B(\text{phényle})_4^-$.

**12.** Amides bis-cationiques selon une ou plusieurs des revendications 1 à 6, caractérisés par la formule I

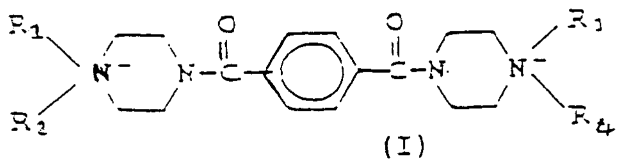

(I)

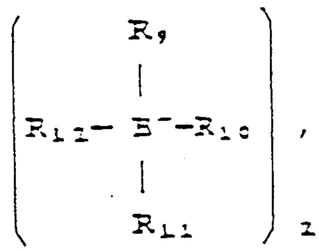

$R_1$ et $R_3$ représentant chacun indépendamment H, $CH_3$ ou $C_2H_5$, $R_2$ et $R_4$ représentant $CH_3$ ou $C_2H_5$, et l'anion $BF_4^-$ ou $B(\text{phényle})_4^-$.

**13.** Procédé de préparation d'amides et imides, de mélanges d'amides et imides ou de cristaux mixtes à anions et/ou cations mixtes, de formules générales I à III selon la revendication 1, procédé caractérisé en ce que l'on fait réagir des amides et imides de formules générales IV à VI

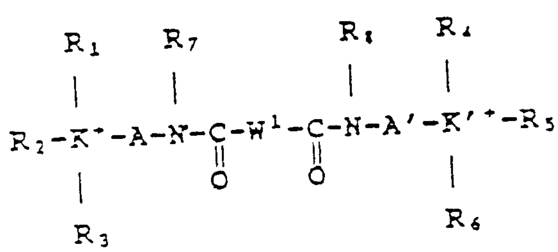

$2\ X^-$,

(IV)

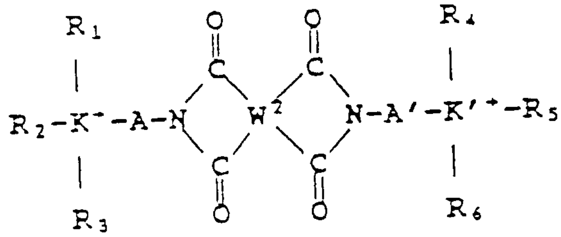

$2\ X^-$,

(V)

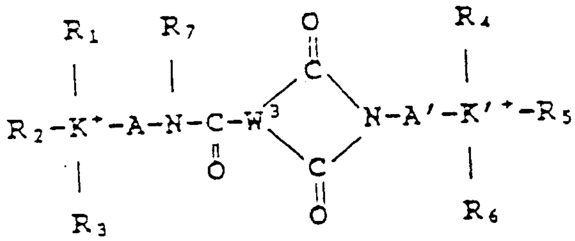

$2\ X^-$,

(VI)

les divers symboles ayant les significatios indiquées aux revendications 1 à 11 et $X^-$ représentant l'équivalent stoechiométrique d'un ou de plusieurs anions halogènes, méthylsulfate, éthylsulfate, hydrogénosulfate ou sulfate, avec un ou plusieurs des borates minéraux indiqués à la revendication 1, aux revendications 6 à 11 et à la revendication 15, dans de l'eau ou dans un mélange d'eau et d'un acide ou d'eau et d'un solvant organique, à des températures d'environ 10 à 90 °C.

**14.** Procédé selon la revendication 13, caractérisé en ce que l'on fait réagir les composés de formule IV à VI indiqués à la revendication 13 dans un mélange d'eau et d'acide acétique, d'isopropanol, d'isobutanol ou de méthylisobutylcétone.

**15.** Procédé selon la revendication 13 et/ou 14, caractérisé en ce que l'on fait réagir les composés de formules IV à VI indiqués à la revendication 13 avec l'un des sels suivants :
tétraphénylborate de sodium,
tétra-o-fluorophénylborate de sodium,
tétra-m-fluorophénylborate de sodium,
tétra-p-fluorophénylborate de sodium,
tétra-o-chlorophénylborate de sodium,
tétra-m-chlorophénylborate de sodium,
tétra-p-chlorophénylborate de sodium,
tétra-o-tolylborate de sodium,
tétra-m-tolylborate de sodium,
tétra-p-tolylborate de sodium,
tétra-1-naphtylborate de sodium,
tétra-2-naphtylborate de sodium,
tétra-o-méthoxyphénylborate de sodium,
tétra-m-méthoxyphénylborate de sodium,
tétra-p-méthoxyphénylborate de sodium,
tétra-o-biphénylborate de sodium,
tétra-m-biphénylborate de sodium,
tétra-p-biphénylborate de sodium,
tétra-benzylborate de sodium,

tétra-o-pyridylborate de sodium,
tétra-m-pyridylborate de sodium,
tétra-p-pyridylborate de sodium ou
tétrafluoroborate de sodium.